# EUROPEAN PATENT SPECIFICATION

(11) **EP 3 104 796 B1**
(45) Date of publication and mention of the grant of the patent: **10.04.2019**
(21) Application number: 15749070.7
(22) Date of filing: 12.02.2015
(51) Int. Cl.: A61F 7/10, A61B 18/02

(54) **METHOD AND APPARATUS FOR AFFECTING PIGMENTATION OF TISSUE**
VERFAHREN UND VORRICHTUNG ZUR BEEINFLUSSUNG DER PIGMENTIERUNG VON GEWEBE
PROCÉDÉ ET APPAREIL POUR AFFECTER UNE PIGMENTATION DE TISSU

(30) Priority: 12.02.2014 US 201461939162 P
(43) Date of publication of application: 21.12.2016
(60) Divisional application: 19158790.6
(73) Proprietor: The General Hospital Corporation, Boston, MA 02114 (US); Blossoms Innovations, Llc, Boston, Massachusetts 02114 (US)
(72) Inventor: ANDERSON, Richard Rox, Boston, Massachusetts 02114 (US); MANSTEIN, Dieter, Coral Gables, Florida 33143 (US); CHAN, Henry Hin Lee, Hong Kong (CN); ZUO, Vincent, Boston, Massachusetts 02114 (US); TING, Joseph, Framing, Massachusetts 01702 (US)
(74) Representative: LKGLOBAL Lorenz & Kopf PartG mbB Patentanwälte
(86) International application number: PCT/US2015/015626
(87) International publication number: WO 2015/123420

(56) References cited:
- WO-A1-2013/075006
- WO-A1-2013/075006
- US-A1- 2003 100 936
- US-A1- 2011 060 322
- US-A1- 2011 313 411
- US-A1- 2011 313 411
- US-A1- 2012 022 518

## Description

### FIELD OF THE DISCLOSURE

The present disclosure relates generally to affecting pigmentation of biological tissue, and more specifically to method and apparatus for controlled cooling of skin tissue to affect the pigmentation thereof.

### BACKGROUND

Controlled cooling or freezing of biological tissue, such as skin tissue, can produce various effects. Certain tissue freezing procedures and devices, such as conventional cryoprobes, can cause severe freezing of tissue and generate cellular damage. For example, 15 moderate degrees of cooling or freezing can produce particular effects, such as affecting the expression of skin pigmentation.

There is a demand for cosmetic products that can lighten the appearance of skin or otherwise controllably affect skin pigmentation. For example, it may be desirable to lighten the overall complexion or color of a region of skin to alter the general appearance for cosmetic reasons. Also, lightening of the appearance of certain hyperpigmented regions of skin, such as large freckles, 'café au lait' spots, melasma, or dark circles under the eyes that may result from excessive local amounts of pigment in the skin, may also be desirable for cosmetic reasons. Hyperpigmentation can result from a variety of factors such as UV exposure, aging, stress, trauma, inflammation, etc. Such factors can lead to an excess production of melanin, or melanogenesis, in the skin by melanocytes, which can lead to formation of hyperpigmented areas. Such hyperpigmented areas can be typically located within the epidermis. However, they can also result from excess melanin deposited within the dermis. Many topical formulations are being marketed that claim to lighten age spots and reduce the effects of such hyperpigmentation. However, most of these cosmetic formulations have questionable effectiveness, and may produce unwanted side effects.

Hypopigmentation of skin tissue has been observed in response to temporary cooling or freezing of the tissue, such as may occur during cryosurgery procedures. Loss of pigmentation following skin cooling or freezing may result from decreased melanosomes production, destruction of melanocytes, or inhibited transfer of melanosome into the keratinocytes in the lower region of the epidermal layer. The resultant hypopigmentation may be long-lasting or permanent. It has also been observed that some of these freezing procedures can generate regions of hyperpigmentation of skin tissue.

It has been recognized recently that controlled cooling or freezing of skin tissue can generate hypopigmentation as described, e. g., in US. Patent Publication No. 2011/0313411. Factors that can affect the pigmentation outcomes can include cooling temperature, cooling time, and geometric features of a cooled object that may be contacted with the skin surface to cool it. Surfaces geometries that have been proposed for such cooling or freezing include, e. g., simple flat plates, plates with small protrusions (e.g., less than about 1-2 mm in diameter), and plates with discontinuous raised features (e.g., greater than about 5-10 mm in diameter). There may be some variability in pigmentation reduction and consistency in such procedures based on the use of different cooled plate geometries, where such results may further be sensitive to other parameters such as plate temperature and contact duration.

US 2011/313411 describes method and apparatus for effecting (e.g., lightening) an appearance of skin by cooling or freezing small separated surface regions of the skin to produce regions of local hypopigmentation. The width of the regions can be, for example, smaller than about 1 mm or 0.5 mm, and a distance between these frozen regions can be greater than about 3 times the width of the regions. An apparatus may include a plurality of spatially-separated thermally conductive arrangements that can be affixed or otherwise coupled to a base. The conductive arrangements can be cooled and then contacted with the skin surface to produce small regions of hypopigmentation.

US 2012/022518 describes systems and methods that enable delivery of radio frequency energy and cryotherapy applications to adipose tissue for reduction and contouring of body fat.

WO 2013/075006 describes cosmetic method and apparatus that can provide cooling and/or freezing of skin tissue proximal to the skin surface to generate an appearance of lightening or reduced pigmentation in the skin. The skin can be cooled to a temperature of less than about -5 degrees Celsius for a duration of about one minute or less, using a plurality of cooled contact surfaces, each having a width between about 2 mm and about 20 mm. A cooling arrangement can be provided to provide controlled heat removal from the skin tissue being treated. A sensor can optionally be provided to detect freezing of tissue proximal to the cooled surfaces.

Accordingly, there may be a need for methods and apparatus that can provide controlled freezing of skin or other tissue, and gradual lightening of skin tissue that can address and/or overcome at least some of the deficiencies or issues described herein above.

### SUMMARY OF EXEMPLARY EMBODIMENTS

The herein described exemplary embodiments pertain to cosmetic method and apparatus that can address and/or overcome at least some of the deficiencies or issues described herein above. Synergetic effects may arise from different combinations of the features and embodiments described herein, although all such combinations might not be described in detail. Further, it shall be noted that all embodiments of the present disclosure concerning a method can be carried out with the order of the steps as described, nevertheless this may not be the only and essential order of the steps of the exemplary methods. For example, all different orders and combinations of the method steps and procedures are herewith described.

The invention is defined by the independent claims. Preferred embodiments are given by the dependent claims. Exemplary embodiments of the present disclosure relate to non-invasive methods and apparatus for controlled cooling or freezing of skin tissue, which can reduce an overall pigmentation of an area of skin using cryogenic techniques. In one exemplary embodiment of the present disclosure, an apparatus can be provided for controllably cooling or freezing regions of skin at particular temperatures and time durations to produce a lightening of the skin appearance.

According to an exemplary embodiment of the present disclosure, the apparatus can include a cooled contact arrangement, preferably formed at least partially of a material having a large thermal effusivity, e.g., an effusivity at least about 10 times greater than that of skin tissue. For example, the plate can be made at least partially of a metal or alloy, such as brass, gold, silver, copper, aluminum, or the like, diamond or diamond-like carbon, a frozen material, or another material that has a high thermal effusivity, such as diamond. The contact arrangement can have a distal surface configured to contact a surface of a region of skin. Optionally, the contact arrangement can be formed using two or more materials having different thermophysical properties (e.g. different thermal effusivities). In one exemplary embodiment, a layer or film of a second material can be provided on the distal (contact) surface of the contact arrangement.

The distal contact surface of the contact arrangement can be substantially planar, or it can be convex or concave to facilitate contact with a region of skin. The contact surface can be provided with a plurality of dimples, e.g., small recesses that may be substantially round and have a diameter or width that is between about 0.3 mm and about 3 mm, or between about 0.5 mm and 2 mm, or optionally about 1 mm. The depth of the dimples can be between about 0.3 mm and about 2 mm, or between about 0.5 mm and about 1.5 mm, or optionally about 1 mm. The dimples can be provided in a regular pattern (e.g. a rectangular or triangular array) or they may be randomly or semi-randomly distributed over the contact surface.

In further exemplary embodiments of the present disclosure, one or more of the dimples can have an elongate shape, with a width (e.g. smaller dimension) that is between about 0.3 mm and about 3 mm, or between about 0.5 mm and 2 mm, or optionally about 1 mm. The length (e.g. longer dimension) of the elongate dimples can be greater than the corresponding width. The depth of such elongate dimples can be between about 0.3 mm and about 2 mm, or between about 0.5 mm and about 1.5 mm, or optionally about 1 mm. The elongate dimples can be provided in a regular pattern (e.g. with their long axes parallel or perpendicular to one another) or their orientations and/or locations may be randomly or semi-randomly distributed over the contact surface. In one exemplary embodiment of the present disclosure, elongate dimples can be provided as a plurality of substantially parallel grooves that can extend across a portion or substantially the entire width or length of the contact surface.

In still further exemplary embodiments of the present disclosure, dimples having different sizes and/or shapes (e.g. different widths, lengths, and/or depths) can be provided on a single contact surface. Such dimples can be provided in a regular array or randomly or semi-randomly oriented and located.

The edges of the dimples where the dimples meet the distal surface can optionally be rounded or beveled, e.g., to avoid a sharp edge. The interior surface of a dimple can be rounded, cylindrical, or square in profile, or have another shape. The interior surface of a round dimple can be cylindrical or can have the shape of a portion of a sphere or ellipsoid. The interior surface of an elongate dimple can have a shape that is rounded, such as a portion of a circular or ellipsoidal cylinder, or it may be provided with internal corners.

An area fraction of the dimples on the contact surface can be, e.g., between about 0.05 and about 0.50, or optionally between about 0.10 and about 0.30, or about 0.20. Such exemplary ranges and values of fractional area coverage can provide a sufficient area of direct skin contact by the contact surface while also providing sufficient areal density of dimples to improve local cooling and/or freezing efficacy to generate hypopigmentation effects.

In further exemplary embodiments of the present disclosure, a cooling arrangement can be provided in the apparatus. The exemplary cooling arrangement can include, e.g., a reservoir provided in thermal contact with the cooling plate. A refrigerant or other heat-absorbing medium, such as, e.g., a saline solution, a water-alcohol mixture, a water-glycol mixture, or the like can be provided in the reservoir to cool the plate. Optionally, a cooled refrigerant can be circulated through the reservoir and/or one or more ducts formed in the reservoir to provide continuous cooling to the plate. A control arrangement can optionally be provided, and the cooling arrangement can be configured to control and/or maintain a particular temperature of the cooling plate while the cooling plate is in contact with the skin. One or more temperature sensors can be provided on or in the contact element to facilitate temperature control of portions of the contact element.

In another exemplary embodiment of the present disclosure, a thermoelectric cooling device such as, e.g., a Peltier device, or other cooling source can be provided in thermal contact with the contact arrangement to cool it. In certain embodiments, the contact arrangement can be formed as part of a cooled portion of the cooling device and configured to be placed directly on the skin surface.

One or more temperature and/or optical sensors, or other types of sensors, can be provided to control the temperature of the cooling plate or device, to detect the local temperature of contacted/cooled tissue, and/or to detect local freezing of skin tissue while the cooling plate or device is placed in contact with the skin surface. Treatment time can optionally be determined relative to the initiation of freezing, e.g., as a duration of contact time between the cold object and the skin surface after local tissue freezing has begun. Temperature can be measured using contact sensors, non-contacting sensors, or both. A heating arrangement can optionally be provided to warm the frozen tissue after the particular treatment time has elapsed. A feedback signal can be generated and transmitted to the cooling device or the control arrangement, if present, such that undesired or excessive cooling or freezing can be avoided. For example, a feedback control can be provided to facilitate a safe and effective treatment procedure for which no danger or risk results for the person undergoing the treatment.

According to further exemplary embodiments of the present disclosure, a method and apparatus for detection of tissue freezing at the onset of such freezing can be provided. Such exemplary freezing detection can be based on, e.g., temperature detection, optical detection, and/or measurement of electrical and/or mechanical impedance of the skin tissue.

In another exemplary embodiment of the present disclosure, one or more pressure sensors can be provided to detect a contact pressure between the contact surface of the apparatus and the skin surface.

In yet another exemplary embodiment of the present disclosure, the contact arrangement can include a flexible or pliable contact surface to facilitate improved contact between the contact surface of the apparatus and the skin surface. A pliable or malleable substance having a high thermal conductivity or effusivity can be provided between the cooling arrangement and a flexible film at the contact surface to facilitate a deformation of the contact surface, while maintaining a high rate of heat extraction from the skin tissue. Such pliable or malleable substance can include, e.g., a thermally conductive paste or gel, or a two-phase slush or slurry having a phase transition temperature or range that is at, close to, or in proximity to, a predetermined contact temperature.

In still another exemplary embodiment of the present disclosure, a vacuum arrangement comprising one or more channels in the contact arrangement can be provided to facilitate an improved contact between the contact surface of the apparatus, e.g., within the dimples, and the skin surface.

In other exemplary embodiments of the present disclosure, the cooling apparatus can be provided with a plurality of cooling arrangements and/or a plurality of contact arrangements. One or more temperature sensors, pressure sensors, freezing detectors, and/or control arrangements can optionally be associated or provided in communication with each cooling arrangement and/or with each contact arrangement.

In a further exemplary embodiment of the present disclosure, a cosmetic method can be provided to produce hypopigmentation in skin tissue. In one exemplary embodiment, the method for lightening the appearance of skin can be provided that includes cooling and/or freezing a region of skin to induce localized hypopigmentation effects. Further lightening can be achieved by treating a particular region of skin more than once.

In a still further exemplary embodiment of the present disclosure, the cosmetic method can include freezing regions of skin tissue, for example, to at least the depth of the basal layer, e.g., to at least about the depth of the dermal-epidermal junction, which can provide a hypopigmentation effect. This exemplary freezing can preferably be achieved by contacting the skin surface with the distal surface of a contact arrangement, e.g., a cooling plate or the like, that is provided with a plurality of dimples and that is provided at a temperature colder than about -5 degrees Celsius, e.g., between about -7 and -10 degrees Celsius. In certain exemplary embodiments, temperatures as low as about -15 to -20 degrees Celsius can be used. Cooling or treatment times, which can correspond to the duration that the cold object is contacted with the skin surface after local tissue freezing begins, can be less than about two minutes, or preferably less than about one minute, e.g., between about 30 seconds and 1 minute when the temperature of the distal surface of the contact arrangement is between about -7 and -10 degrees Celsius. Shorter cooling times can be used when the distal surface of the contact arrangement is provided at colder temperatures, e.g. less than about 30 seconds or even less than about 15 seconds when the temperature of the cold object is between about -15 to -20 degrees Celsius. Such temperatures and times can lead to hypopigmentation responses in skin tissue while being fast enough to facilitate sequential cooling or freezing of a plurality of regions of skin.

One or more temperature and/or optical sensors, or other types of sensors, can be provided to control temperature of the contact arrangement and/or of a cooling device provided in thermal communication with the contact arrangement, to detect the local temperature of contacted/cooled tissue, and/or to detect local freezing of skin tissue while the contact arrangement is placed in contact with the skin surface. Treatment time(s) can optionally be determined relative to the initiation of freezing, e.g., as a duration of contact time between the cold object and the skin surface after local tissue freezing has begun. Temperature can be measured using contact sensors, non-contacting sensors, or both. A heating arrangement can optionally be provided to warm the frozen tissue after the particular treatment time has elapsed. A feedback signal may be generated and sent to the cooling device, if present, such that undesired or excessive cooling or freezing is avoided. In other words, a feedback control can be provided to ensure a safe treatment procedure for which no danger or risk results for the person undergoing the treatment.

The exemplary method and apparatus according to an exemplary embodiment of the present disclosure can provide gradual lightening of the skin area being treated by controllably forming depigmented areas. Multiple treatments can be used to lighten the area further. The exemplary methods and apparatus described herein may also improve the general appearance of the skin by stimulating a response in the small regions of frozen tissue.

The exemplary described cosmetic method has been tested, and is determined to be a safe and routine procedure that can be implemented in beauty parlors or other settings. The exemplary method can be a non-invasive a method. Moreover, the method can be safe as it is non-invasive, does not present a substantial health risk, and does not require professional medical expertise to be performed. No clinician is needed to perform the embodiments of the method described herein, and no risk, much less a health risk, is presented for a person being treated with said cosmetic method, as will become clear from the following description.

These and other objects, features and advantages of the present disclosure will become apparent upon reading the following detailed description of embodiments of the disclosure, when taken in conjunction with the appended claims.

### BRIEF DESCRIPTION OF THE DRAWINGS

Further objects, features and advantages of the present disclosure will become apparent from the following detailed description taken in conjunction with the accompanying figures showing illustrative embodiments, results and/or features of the exemplary embodiments of the present disclosure, in which:
FIG. 1 is a side cross-sectional view of an exemplary apparatus that can be used to produce a hypopigmentation cryogenically in a skin tissue according to an exemplary embodiment of the present disclosure;
FIG. 2A is an exemplary cross-sectional side view of a contact element of the exemplary apparatus shown in FIG. 1;
FIG. 2B is a bottom view of a first exemplary configuration of the contact element shown in FIG. 2A;
FIG. 2C is a bottom view of a second exemplary configuration of the contact element shown in FIG. 2A;
FIG. 2D is a bottom view of a third exemplary configuration of the contact element shown in FIG. 2A;
FIG. 2E is a bottom view of a fourth exemplary configuration of the contact element shown in FIG. 2A;
FIG. 2F is a bottom view of a fifth exemplary configuration of the contact element shown in FIG. 2A;
FIG. 2G is a bottom view of a sixth exemplary configuration of the contact element shown in FIG. 2A;
FIG. 2H is a bottom view of a seventh exemplary configuration of the contact element shown in FIG. 2A;
FIG. 2I is a bottom view of an eighth exemplary configuration of the contact element shown in FIG. 2A;
FIG. 2J is a bottom view of a ninth exemplary configuration of the contact element shown in FIG. 2A;
FIG. 2K is a bottom view of a tenth exemplary configuration of the contact element shown in FIG. 2A;
FIG. 3A is a side cross-sectional view of an exemplary contact element containing a rounded dimple contacting a skin surface according to an exemplary embodiment of the present disclosure;
FIG. 3B is a side cross-sectional view of the exemplary contact element containing a dimple having a cylindrical or rectangular profile according to the present disclosure;
FIG. 3C is a side cross-sectional view of the exemplary contact element containing a shallow rounded according to another exemplary embodiment of the present disclosure;
FIG. 3D is a side cross-sectional view of the exemplary contact element containing a deep dimple according to yet an exemplary embodiment of the present disclosure;
FIG. 4A is a bottom view of an exemplary configuration of the contact element shown in FIG. 2A that includes a plurality of temperature sensors;
FIG. 4B is a cross-sectional side view of an exemplary contact element that includes 3 thermal sensors provided at different locations on and within the contact element;
FIG. 5 is a side cross-sectional view of an exemplary apparatus for controllably cooling skin tissue that includes an optical arrangement to detect local skin freezing according to an exemplary embodiment of the present disclosure;
FIG. 6 is an exemplary data graph generated with an apparatus according to an exemplary embodiment of the present disclosure illustrating changes in both measured surface temperature and optical reflectance when a region of live pig skin is cooled and local freezing of the skin commences;
FIG. 7 is a cross-sectional side view of the exemplary contact element that includes two different materials having different thermophysical properties according to an additional exemplary embodiment of the present disclosure;
FIG. 8 is a side cross-sectional view of an exemplary apparatus for controllably cooling or freezing skin tissue that includes pressure sensors to detect contact pressure of the contact element with the skin surface according to an exemplary embodiment of the present disclosure;
FIG. 9 is a cross-sectional side view of the exemplary contact element that includes a vacuum arrangement configure to facilitate contact between the contact surface and the skin surface according to a yet further exemplary embodiment of the present disclosure;
FIG. 10 is a cross-sectional side view of an exemplary contact element that includes a flexible or pliable contact surface;
FIG. 11 is a side cross-sectional view of the exemplary apparatus for controllably cooling or freezing skin tissue that includes more than one cooling arrangement and more than one contact element according to another exemplary embodiment of the present disclosure;
FIG. 12A is an exemplary image of a region of swine skin obtained 4 weeks after it was cooled to -9 degrees C for a contact time of 15 seconds using a dimpled contact surface according to an exemplary embodiment of the present disclosure;
FIG. 12B is an exemplary image of the region of swine skin shown in FIG. 12A obtained 9.5 weeks after it was cooled;
FIG. 13A is an exemplary image of a region of swine skin obtained 4 weeks after it was cooled to -9 degrees C for a contact time of 15 seconds using a smooth contact surface;
FIG. 13B is an exemplary image of the region of swine skin shown in FIG. 13A obtained 9.5 weeks after it was cooled;
FIG. 14A is an exemplary image of a region of swine skin obtained 4 weeks after it was cooled to -9 degrees C for a contact time of 30 seconds using a dimpled contact surface according to an exemplary embodiment of the present disclosure;
FIG. 14B is an exemplary image of the region of swine skin shown in FIG. 14A obtained 9.5 weeks after it was cooled;
FIG. 15A is an exemplary image of a region of swine skin obtained 4 weeks after it was cooled to -9 degrees C for a contact time of 30 seconds using a smooth contact surface; and
FIG. 15B is an exemplary image of the region of swine skin shown in FIG. 15A obtained 9.5 weeks after it was cooled.

Throughout the drawings, the same reference numerals and characters, unless otherwise stated, are used to denote like features, elements, components, or portions of the illustrated embodiments. Similar features may thus be described by the same reference numerals, which indicate to the skilled reader that exchanges of features between different embodiments can be done unless otherwise explicitly stated. Moreover, while the present disclosure will now be described in detail with reference to the figures, it is done so in connection with the illustrative embodiments and is not limited by the particular embodiments illustrated in the figures. It is intended that changes and modifications can be made to the described embodiments without departing from the scope of the present disclosure as defined by the appended claims.

### DETAILED DESCRIPTION OF EXEMPLARY EMBODIMENTS

According to an exemplary embodiment of the present disclosure, a cryotherapybased approach can be used to controllably and non-invasively cool and/or freeze regions of skin tissue. Such cooling or freezing can lighten the overall appearance of the skin, or reduce the overall darkness of certain skin areas that have excess pigmentation. For example, contacting regions of skin with cold objects can inhibit formation and/or expression of 15 pigment in the underlying skin. This effect can provide long-lasting or possibly permanent lightening of the areas being treated.

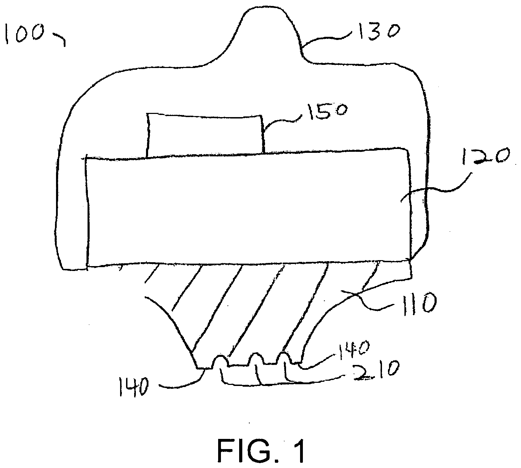

FIG. 1 provides a cross-sectional view of an exemplary apparatus 100 for controllably cooling and/or freezing skin, e.g., to produce hypopigmentation effects in skin tissue, in accordance with exemplary embodiments of the present disclosure. The exemplary apparatus 100 can include a contact element 110 provided in a thermal communication with a cooling arrangement 120. In certain exemplary embodiments, the contact element 110 and the cooling arrangement 120 can be formed at least in part from a single material. A control arrangement 150 can optionally be provided and used to control certain aspects of the cooling arrangement 120, e.g., temperature, timed shutoff, etc. The cooling arrangement 120, control arrangement 150, and/or contact element 110 can optionally be provided within or affixed to a housing or handpiece 130, as shown in FIG. 1, e.g., to facilitate handling and positioning of the apparatus 100. The exemplary apparatus 100 shown in FIG. 1 is not necessarily drawn to scale. For example, the relative dimensions of the cooling arrangement 120 and contact element 110 are not limited to the proportions illustrated in the FIG. 1. In further exemplary embodiments of the present disclosure, the contact element 110 can be larger or smaller in width or cross-sectional area as compared to the dimensions of the cooling arrangement 120.

The contact element 110 can include a distal (contact) surface 140 that is configured to contact a skin surface. The distal surface 140 can be substantially flat. In
further exemplary embodiments of the present disclosure, the distal surface 140 can be convex or concave to better match the local shape of skin tissue being treated and/or to provide good thermal contact with the skin surface when the apparatus 100 is placed on the area of the skin to be treated. In still further exemplary embodiments of the present disclosure, the contact element 110 can be detachable from the cooling arrangement 120, e.g., so that a plurality of contact elements 110 having different sizes, shapes, and/or surface features as described herein can be used with a single cooling element 120.

The distal contact surface 140 can have a large width or diameter configured to contact the surface of a region of skin, e.g., a diameter or width that is greater than about 3 cm, or greater than about 5 cm, to facilitate treatment of large areas of skin. In further embodiments, the width of the distal surface 140 can be small, e.g., on the order of 1-2 cm or less, which may facilitate improved temperature control and/or treatment of particular features on the skin.

The contact element 110 can be formed from a metal or a metal alloy, or another material having a high thermal effusivity, e.g., such that values of these thermophysical properties are greater than the corresponding values for skin tissue. The thermal effusivity ε is equal to the square root of the product of a material's thermal conductivity and its volumetric heat capacity. The thermal effusivity is a measure of the ability of a material to exchange heat with its surroundings and to maintain a consistent temperature as it does so. For example, the interface temperature Tᵢ where two semi-infinite materials at temperature T₁ and T₂, respectively, are brought into contact will depend on their relative effusivities, ε₁ and ε₂, as Tᵢ = T₁ + (T₂-T₁)*[ε₂/(ε₂+ε₁)]. Accordingly, e.g., with ε₂>>ε₁, the interface temperature where the two materials are in contacts will remain close to T₂ as heat flows from one to the other. In this manner, the surface of a first material will be cooled down close to the temperature of a second material having a much higher thermal effusivity when the second material is brought into contact with the first material.

For example, the contact element 110, at least in part or wholly, can be made of brass, copper, silver, aluminum, an aluminum alloy, steel, graphite, diamond, diamond-like carbon, other materials which are used in conventional contact cryoprobes, or combinations thereof. For example, the contact element 110 can be formed, wholly or at least in part, from materials having a much higher thermal conductivity than the skin tissue, and can be used to facilitate an extraction of heat from the portion of the tissue contacted by the distal surface 140 of the contact element 110. Further, materials having a much higher thermal effusivity than the skin tissue, e.g. at least about 10 times the thermal effusivity of skin, can be more readily maintained at a cold temperature. Such high-effusivity materials thereby may extract heat more effectively from the portion of tissue contacted by the contact element 110 than materials having lower thermal effusivities, and facilitate a better control of the tissue temperature at a contact interface..

In certain exemplary embodiments of the present disclosure, the distal contact surface 140 of the contact element 110 can be smaller in area than the proximal end of the contact element 110 that contacts the cooling arrangement 120. Such geometry can provide certain advantages. For example, the narrower or tapered distal end of the contact element 110 can facilitate a more precise placement of the distal surface 140 on a particular location of the skin surface to be cooled, e.g., while reducing visual obstruction by the housing 130. Further, the relatively larger proximal end of the contact element 110 can provide a larger area that can be directly cooled by the cooling arrangement 120 to facilitate increased extraction of heat from the smaller distal contact surface 140. In certain embodiments, the area of the proximal end of the contact arrangement distal contact surface 140 can be at least twice as large as the area of the distal contact surface 140, e.g., 3-5 times as large.

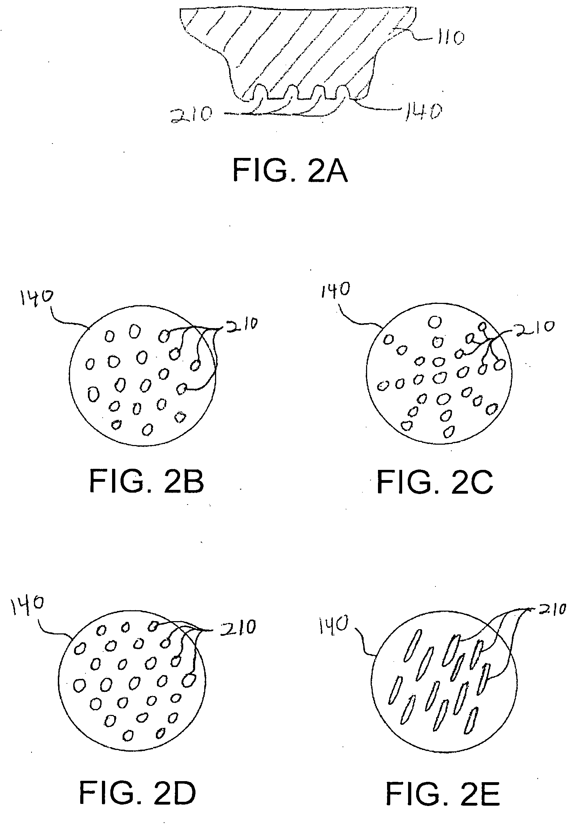

The distal surface 140 of the contact element 110 can be provided with a plurality of dimples 210, e.g., indentations or pockets formed in the contact surface 140 of the contact element 110, as shown in the cross-sectional side view of FIG. 2A. Such dimples 210 can be substantially round and have a diameter or width that is between about 0.3 mm and about 3 mm, or between about 0.5 mm and 2 mm, or optionally about 1 mm. The depth of the dimples can be between about 0.3 mm and about 2 mm, or between about 0.5 mm and about 1.5 mm, or optionally about 1 mm. The edges of the distal surface 140 can be rounded or beveled, as shown in FIG. 2A, which can facilitate continuous contact of the distal surface 140 with the skin surface while avoiding contact with any sharp or abrupt edges or corners when the apparatus 100 is placed against the skin surface for treatment.

An exemplary end view of the contact surface 140 with dimples 210 is shown in FIG. 2B. An area fraction of the dimples 210 on the contact surface 140 can be e.g., between about 0.05 and about 0.50, or optionally between about 0.10 and about 0.30, or about 0.20. Such exemplary ranges and values of fractional area coverage can provides a sufficient area of direct skin contact by the contact surface 140 while also providing sufficient areal density of dimples 210 to improve local cooling and/or freezing efficacy to generate hypopigmentation effects.

Although the size and depth of the exemplary dimples shown in FIGS. 2A and 2B are substantially uniform, individual dimple sizes and/or depths associated with a single contact element 110 can vary within the ranges described herein in further embodiments of the disclosure.

The exemplary arrangement of the dimples 210 on the contact surface 140 can be substantially random, as shown in FIG. 2B. In a further exemplary embodiment of the present disclosure, shown in FIG. 2C, the dimples 210 can be provided in a radial arrangement. Such exemplary arrangement/configuration can yield a lower density of dimples 210 (e.g., a wider average spacing between adjacent dimples 210), which may lead to a reduced effect of the dimples 210 near the perimeter of the contact surface 140. In a still further embodiment, the dimples 210 can be provided in a regular array, e.g., a hexagonal array as shown in FIG. 2D, or a square array.

In further exemplary embodiments of the present disclosure, the dimples 210 can have an elongated shape, as shown in the exemplary configuration in FIG. 2E. Such elongate dimples 210 can have a smaller dimension (e.g., width) that is between about 0.5 mm and about 3 mm, or optionally about 1 mm. A longer dimension (e.g., length) of such elongate dimples 210 can be greater than the width, e.g., twice the width or longer. For example, the exemplary dimples 210 shown in FIG. 2E have a length that is about five times greater than the width. Other exemplary length-to-width ratios can be provided in further exemplary embodiments of the present disclosure. The depth of the elongate dimples 210 can be between about 0.3 mm and about 2 mm, or between about 0.5 mm and about 1.5 mm, or optionally about 1 mm. The edges of the distal surface 140 can be rounded and/or beveled where these dimples 210 meet the contact surface 140, as shown in FIG. 2A.

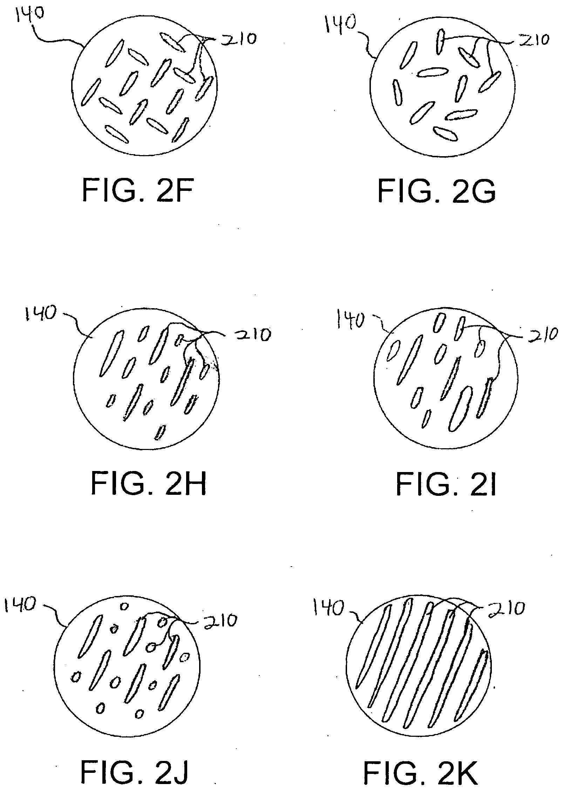

The long axes or dimensions of the exemplary elongate dimples 210 shown in FIG. 2E can be substantially parallel to one another. In a still further exemplary embodiment of the present disclosure, the long axes of some elongate dimples 210 can be substantially perpendicular to other ones, e.g., as shown in FIG. 2F. In still further exemplary embodiments of the present disclosure, the long axes of the elongate dimples 210 can be provided at various angles to one another on the contact surface 140. The elongate dimples 210 can be provided in a regular array or pattern, as shown in FIGS. 2E and 2F. Alternatively or in addition, the elongate dimples 210 can be provided in a non-uniform or random arrangement, as shown, e.g., in FIG. 2G.

In further exemplary embodiments of the present disclosure, individual ones of the dimples 210 provided on a single distal contact surface 140 can have different sizes, shapes, and/or orientations. For example, different ones of the elongate dimples 210 can have the same width (small dimension) and different aspect ratios (e.g., ratios of length to width) as shown, e.g., in FIG. 2H. In further exemplary embodiments of the present disclosure, different ones of the dimples 210 can have different widths and/or different lengths from one another as shown, e.g., in FIG. 2I. In still further exemplary embodiments of the present disclosure, the contact surface 140 can include both round and elongate dimples 210 as shown, e.g., in FIG. 2J. In general, it can be preferable that the width (or diameter) and depth of the various dimples 210 are within the size ranges described herein. In yet another exemplary embodiment of the present disclosure, elongate dimples 210 can be provided as a plurality of substantially parallel grooves, as shown, e.g., in FIG. 2K. The ends of such dimples 210 can lie within the perimeter of the contact surface 140 as shown in the exemplary configuration of FIG. 2K. Alternatively, the elongate dimples 210 can extend through the perimeter of the contact surface 210, such that at least some of the dimples 210 form continuous grooves that span the full length of the contact surface 140.

The interior surface of a dimple 210 can be rounded, cylindrical, or square in profile, or have another shape. For example, the interior surface of a round dimple 210 can be cylindrical or can have the shape of a portion of a sphere or an ellipsoid. The interior surface of an elongate dimple 210 can have a shape that is rounded, such as a portion of a circular or ellipsoidal cylinder, or it may be provided with internal corners, e.g., as a squared-off channel or the like.

In general, a nearest distance between adjacent ones of the dimples 210 at the contact surface can be at least as large as the width of the dimples 210. This exemplary distance between adjacent dimples 210 can be greater than their width, e.g., as shown in FIGS. 2B-2K. Such separation distances can facilitate sufficient heat extraction from the vicinity of each dimple 210 and provide a sufficient area of the contact surface 140 between dimples 210 so the contact surface 140 can be placed comfortably against the skin surface.

The shape of the exemplary contact surface 140 shown in FIGS. 2B-2K is substantially round. In a further exemplary embodiment of the present disclosure, the contact surface 140 can be provided with a shape that is substantially square, rectangular, or hexagonal. Such shapes can facilitate treatment of larger areas of skin by successively contacting adjacent areas thereof with the contact surface 140 while reducing or avoiding significant overlap in treated areas. In further embodiments, the contact surface 140 can have still different shapes.

The aspect ratio of the contact surface shape can be varied in different exemplary embodiments. For example, a square, rectangular or hexagonal shape of the contact surface 140 can facilitate uniform coverage of a larger area of skin tissue by sequential placement of the apparatus 100 on adjacent regions of skin tissue, such that substantially all of the desired treatment area of skin has been cooled by the apparatus 100 with little or no overlap of such treatment regions. Other exemplary plate shapes and/or sizes can also be provided, e.g., to conform to particular regions of skin and/or to conform to a shape of a particular skin feature to be treated such as, e.g., an age spot or the like.

One or more of any of the exemplary dimple shapes, dimensions, dimple patterns, contact surface sizes and shapes, etc., or combinations thereof, can be used with any of the exemplary embodiments and features of the present disclosure. For example, a single contact surface 140 can include a plurality of dimple shapes (e.g. round, elongated, etc.), spatial arrangements, etc., and certain various ones of such dimples 210 provided on a single contact surface 140 can have one or more characteristic diameters, widths and/or depths, as described herein.

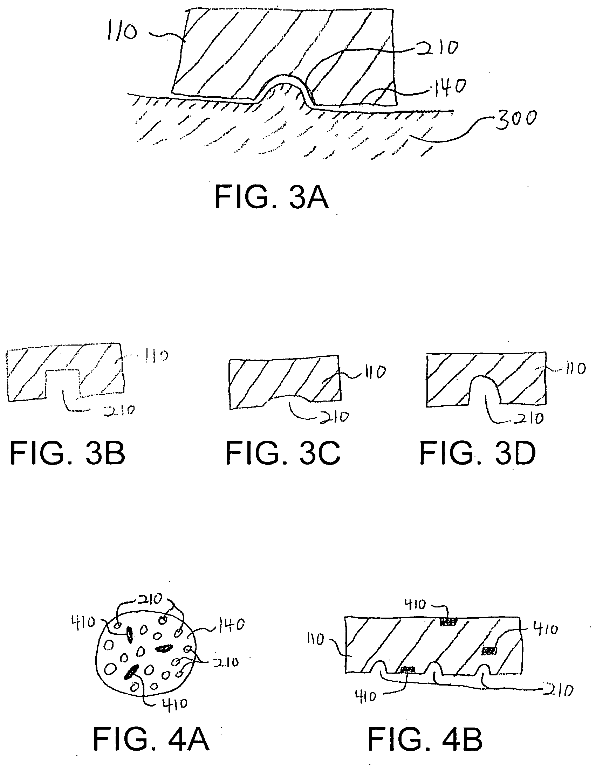

The exemplary ranges and values of geometrical parameters for the dimples 210 can provide partial or full contact between the skin surface and the inner surface of the dimples 210 when the contact surface 140 is placed against the skin. This can improve local cooling and/or freezing efficacy to generate hypopigmentation effects as described in further detail herein. For example, a cross-sectional view of a contact element 110 containing a dimple 210 that is placed against skin tissue 300 is shown in FIG. 3A. When the contact surface 140 is placed against the skin surface, a small portion of the pliable skin 300 can protrude into the dimple 210, as illustrated in FIG. 3A.

Without being bound to a particular theory, such local deformation of the skin 300 can provide an increased local surface area that is in contact with the inner surface of the dimple 210. Some local stretching of the skin 300 where the edges of the dimple 210 meet the contact surface 140 can also occur. Further, heat can be extracted sideways from a portion of the skin 300 within the dimple 210 through the interior surface of the dimple 210, which can enhance the local effectiveness of skin cooling. Because of the relatively small width of the dimple 210, as described herein, such enhanced cooling can be localized to the upper portion of the skin 300 (e.g., the epidermal region) within the dimple 210. The thermally conductive material of the contact element 110 between the dimples 210 can also provide better heat extraction from the skin 300 than, e.g., a cold object, made of the same material and provided at the same temperature, containing a plurality of discrete small protrusions contacting the skin.

Accordingly, for a particular temperature provided or present at the contact surface 140 (which is generally below 0 degrees C), enhanced local cooling effects as suggested herein may promote local freezing of skin tissue 300 at a dimple 210 as compared to a contact surface 140 at the same temperature without dimples 210. Such factors which can be present in a dimpled contact surface may provide more consistent and/or better hypopigmentation effects than contact surfaces 140 that are substantially flat or which contain one or more protrusions. Because of the suggested enhanced local cooling effect of the dimples 210, exemplary embodiments of the exemplary apparatus 100 described herein can also provide hypopigmentation effects at slightly warmer surface temperatures (e.g., possibly 1-2 degrees warmer) and/or slightly shorter contact times than the temperatures and times needed to produce similar effects using a contact surface 140 without any dimples 210.

The cross-sectional view of the exemplary dimple 210 shown in FIG. 3A can correspond to a substantially hemispherical dimple or, alternatively, a cylindrical groove that can extend into a plane of the page, or an ellipsoidal dimple. Further exemplary dimple configurations that can be used in accordance with further exemplary embodiments of the present disclosure are shown in FIGS. 3B-3D. For example, the cross-sectional view of the exemplary dimple 210 shown in FIG. 3B can correspond to, e.g., a cylindrical dimple (e.g., one having a circular cross-sectional shape in an orthogonal plane), or, in an example not according to the invention, a rectangular groove. The cross-sectional view of the exemplary dimple 210 shown in FIG. 3C can correspond to, e.g., a shallow round dimple or a shallow cylindrical groove, whereas the exemplary "deep" dimple 210 shown in FIG. 3D can correspond to, e.g., a deeper round dimple or a deep and narrow cylindrical groove. Any of these exemplary dimple shapes, or combinations thereof, can be used in various exemplary embodiments of the present disclosure.

In a further exemplary embodiment of the present disclosure, the cooling arrangement 120 can include a thermoelectric cooling device, e.g., a Peltier device or the like. The cold side of such cooling device 120 can be provided in thermal communication with the contact element 110. A power source for powering such cooling arrangement 120 can be provided as part of the exemplary apparatus 100, or alternatively an external power source can be provided separate therefrom. The hot side of such cooling device 120 can optionally be cooled by contacting it with a cooled object, by allowing or directing air or another gas to flow over at least a portion of it, and/or by other conventional cooling or ventilation techniques. In certain exemplary embodiments, a conventional cold-water circulating arrangement (not shown) can be provided in thermal communication with the cooling device 120 to cool the hot side. In further exemplary embodiments, a conventional heat sink arrangement can be provided to facilitate dissipation of heat from the cooling arrangement 120.

In still further exemplary embodiments of the present disclosure, one or more temperature sensors 410 can optionally be provided within or adjacent to the contact element 110. For example, a contact surface 140 with an exemplary configuration of three thermal sensors 410 provided thereon is shown in FIG. 4A.

A temperature sensor 410 can be provided at one or more locations of the contact element 110. For example, FIG. 4B shows a cross-sectional view of the temperature sensor 410 on or adjacent to the contact surface 140, another temperature sensor 410 provided in a central portion of the contact element 110, and a further temperature sensor 410 provided at a proximal portion of the contact element 110, e.g., adjacent to or close to the cooling arrangement 120 (not shown). For example, the temperature sensor 410 can be provided in a small groove or recess located on the distal surface 140, as shown in the lower portion of FIG. 4B, such that a surface or contact portion of the temperature sensor 410 is substantially co-planar with the contact surface 140. In certain exemplary embodiments, a plurality of temperature sensors 410 can be provided within or adjacent to the contact element 110, which can facilitate a more precise temperature control during operation of the exemplary apparatus 100.

The temperature sensor 410 can include, e.g., one or more thermocouples, thermistors, resistance temperature detectors (RTDs), or the like. A single apparatus 100 can include one or more types of such temperature sensors 410. A temperature sensor 410 having a small size and/or small mass, e.g., a thermistor, may be desirable in certain exemplary embodiments because it can have a smaller effect on the heat transfer and thermal characteristics of the surrounding contact element material. The temperature sensors 410 can be used with any of the exemplary embodiments described herein.

The temperature sensor(s) 410 can be provided in communication with the control arrangement 150 (shown in FIG. 1) that can be associated with the cooling arrangement 120. Such exemplary control arrangement 150 can be provided within the housing 130 as shown in FIG. 1, optionally integrated with the cooling arrangement 120, or it can be provided external to the housing 130. The control arrangement 150 can be configured to alter or control certain parameters of the cooling arrangement 120 such as, e.g., a power level provided to a thermoelectric cooler, a coolant flow rate or coolant temperature that may be used with or part of the cooling arrangement 120 (e.g., to cool the hot side of a thermoelectric cooling device or to provide direct cooling of the contact element 110), etc. For example, a control arrangement 150 that uses a conventional feedback control system can be provided that uses signals from the temperature sensor 410 to facilitate stabilization and/or maintenance of a particular temperature (or range of temperatures) at the contact surface 140 and/or at another location within the contact element 110. The control arrangement 150 can be configured to use a conventional proportional, integral, and/or derivative feedback algorithm to control the operation of the cooling arrangement 120, e.g., to maintain the contact surface 140 of the contact element 110 at a predetermined temperature or to approximate a predetermined temperature profile over time. Such control algorithms are known in the art.

In further exemplary embodiments of the present disclosure, such temperature and process control can be achieved using a plurality of temperature sensors 410. Temperature sensors 410 can be provided on (or coplanar with) the contact surface 140, e.g., near the contact surface 140, within the contact element 110, and/or near or on the proximal end of the contact element 110 (e.g., adjacent to the cooling arrangement 120), as shown in FIG. 4B. Control of the temperature at or near the contact surface 140 can optionally be based at least in part on a temperature measured away from such location by calibrating the thermal response for a particular cooling arrangement 120 and characteristics of a particular contact element 110 (e.g., the shape and material of the contact element 110).

For example, cooling the basal layer of the skin (e.g., proximal to the dermal-epidermal junction) can be achieved by cooling a surface of the skin to a particular temperature for a particular treatment time (which can be determined as a time interval that begins when local freezing of tissue begins). In addition, such cooling or local freezing can produce cosmetically desirable depigmentation effects, e.g., a general lightened appearance of the cooled region of skin. Such lightened appearance may persist for several months or longer. It has also been observed that cooling or freezing the skin surface with a cold contact surface 140 that includes a plurality of dimples 210 thereon, as described herein, can provide more consistent and uniform lightening of the cooled skin area as compared to a comparable cooling procedure provided by a contact surface that is substantially flat or featureless, or that includes a plurality of protrusions thereon.

Accordingly, any of the exemplary embodiments described herein can be configured to provide one or more surfaces (e.g. the contact element 110 and/or the contact surface 140 thereof) at a temperature of about -4 degrees Celsius or colder, e.g., between about -5 degrees and -10 degrees Celsius. Treatment times corresponding to such temperatures can be, e.g., about one minute or less at such temperatures, for example, between about 30 seconds to one minute. Such combinations of temperatures and times that generate freezing in tissue can produce hypopigmentation effects in skin without producing depigmentation (i.e., total pigmentation loss).

In further exemplary embodiments of the present disclosure, the temperature of at least a portion of the contact element 110 can be colder than -10 degrees Celsius, e.g., as cold as -15 or -20 degrees Celsius, with correspondingly shorter contact or treatment times, to generate sufficient local cooling and freezing of the tissue without inducing undesirable tissue damage from excessive cooling. For example, contact times at these colder temperatures can be as short as 30 seconds or less, e.g., about 15-30 seconds, at temperatures of between about -15 and -20 degrees Celsius. Such colder temperatures can be used, for example, to facilitate faster treatment times, and/or to compensate for such effects as thermal inefficiencies in the heat conduction through the contact element 110 and/or warming effects of the skin that can be heated by local blood flow. However, such colder temperatures and corresponding shorter contact times may be more difficult to control precisely and may lead to some excessive local cooling or freezing of tissue, which can result in non-uniform pigmentation effects, some edema or scab formation, etc.

In any of the embodiments described herein, the temperature sensor(s) 410, and/or dimples 210 can be provided directly on the cold surface of the cooling arrangement 120 (e.g., the cold side of a thermoelectric cooling device or the like) if no intermediary contact element 110 is provided, such that the cold surface of the cooling arrangement 120 can also be the contact surface 140.

The temperature of the epidermal layer of the skin tissue contacted by the contact surface 140 of the contact element 110 can preferably be low enough to locally freeze at least a portion of the skin surface region when the exemplary apparatus 100 is brought into contact with the area of the skin to be treated. The contact surface 140 can be placed in contact with the region of the skin being treated for an exemplary duration of time sufficient to generate subsequent hypopigmentation of the frozen regions. This exemplary time duration can be determined as a time interval following initial contact of the apparatus 100 with the skin, or alternatively as a time interval following initiation of local freezing of the skin tissue. Inducing local freezing of the upper layers of skin tissue (e.g., down to the basal layer) at these temperatures can also improve the hypopigmentation response. At least some portions of the upper dermal layer can also be frozen. This exemplary time duration can be greater than about 15 seconds.

The treatment time can preferably be not so long as to cause excessive freezing and issue damage in the cooled or frozen tissue. For example, if the bottom surface of the contact element 110 is maintained at a temperature of between about -4 and -10 degrees Celsius, a contact time between about 30 and 60 seconds can be sufficient to induce local freezing of the skin surface region that can lead to hypopigmentation. The consistency and predictability of such freezing can be improved by the presence of the dimples 210 provided on the contact surface 140, as described herein. In general, an appropriate time of contact can be determined based on the geometry, materials, and initial cooled temperature of the exemplary embodiments of the apparatus described herein. The time and temperature used can be selected to produce a zone of frozen tissue proximal to the contact element 110 for a particular duration.

In certain exemplary embodiments of the present disclosure, the material of the contact element 110 can be selected such that the corresponding apparatus is initially cooled, and the contact element 110 may gradually warm up during prolonged contact with skin tissue. For example, the exemplary apparatus 100 can be placed in a freezer to cool the contact element 110 and the cooling arrangement 120 to a particular temperature. Alternatively, the contact element 110 and/or the cooling arrangement 120 can be cooled by spraying with a refrigerant such as liquid nitrogen, or immersion them in a cold bath, such a chilled solution of alcohol and water or a cold saline solution. Preferably, the bath can be maintained at a predetermined temperature. After cooling, the bottom surface of the contact element 110 can then be pressed against an area of skin to be lightened for a predetermined time, which can locally chill and/or freeze a portion of skin tissue. For example, the contact element 110 and the cooling arrangement 120 can preferably be cooled to at least -4 degrees Celsius or colder, e.g., between -5 or -7 degrees and -10 degrees Celsius, or even as cold as -15 to -20 degrees Celsius, so that the contact element 110 can cool skin tissue sufficiently and freeze at least a portion of the skin tissue when the apparatus 100 is brought into contact with the skin surface, which can also produce a hypopigmentation response. Accordingly, the contact element 110 can be provided in contact with the skin for longer periods of time, and gradual warming of the contact element 110 can prevent excessive cooling or freezing of the regions of skin proximal thereto.

In a further exemplary embodiment of the present disclosure, the exemplary cooling arrangement 120 can include a thermally conductive block of material with one or more channels provided therethrough. A cooled refrigerant can be circulated through the channels to cool the cooling arrangement 120 and the adjacent contact element 110, and optionally to maintain them at a particular temperature. For example, a conventional fluid pump (not shown) can be used to circulate a cooling medium through the channels in such cooling arrangement 120. Such exemplary pump can be located near to or remotely from the apparatus 100, or in certain exemplary embodiments it can be affixed to the exemplary apparatus 100. The cooling medium can also be provided in a reservoir (not shown) that can be insulated and/or actively cooled using conventional techniques. A desired temperature of the contact surface 140 can be maintained, e.g., by controlling or varying a temperature and/or flow rate of the cooling medium based on signals provided by one or more temperature sensors 410 as described herein.

In a still further exemplary embodiment of the present disclosure, the cooling arrangement 120 of the apparatus 100 can include a hollow reservoir (not shown) that can contain a refrigerant that can be selected such that it exhibits a solid-liquid phase change at a particular temperature or a temperature range, for example, at least as cold as about -4 degrees Celsius, e.g., about -5 to -10 degrees Celsius, or optionally as cold as about -15 to - 20 degrees Celsius. For example, when the contact element 110 warms up to the phase change temperature (if it is initially colder), e.g., by contacting warmer skin tissue, the phase-change refrigerant can maintain the temperature of the cooling arrangement 120 and/or the contact element 110 at approximately the phase change temperature or within a particular phase change temperature range for an extended period of time (e.g., during longer contact periods between the contact element 110 and the skin tissue being treated, or during application of the exemplary apparatus 100 to a plurality of regions of skin being treated). Heat extracted from the skin tissue can be conducted through the contact element 110 and the cooling arrangement 120 to the refrigerant provided therein, which can absorb the heat at a relatively constant temperature as the phase change in the refrigerant progresses. This exemplary arrangement can facilitate a predictable and repeatable temperature of the contact surface 140 and corresponding rate of heat transfer flow from the skin contacting the contact element 110 because the temperature difference provided by the apparatus 100 between the cooling arrangement 120 and the contact surface 140 can be maintained substantially constant over extended periods of time. This exemplary arrangement can also provide cooling and/or freezing of the upper portion of the skin tissue at a particular temperature or within a narrow range of temperatures, e.g., about -4 degrees Celsius or colder, e.g., between -5 degrees and -10 degrees Celsius. The duration over which such cooled temperature can be consistently maintained can be based on such factors as, e.g., the size and material of the cooling arrangement 120, and the amount and composition of the phase-change refrigerant provided therein.

Hypopigmentation effects based on contact cooling of the skin surface, as described herein with respect to the various exemplary embodiments, may be more effective if at least a local volume of tissue proximal to the surface is frozen. Cooling of the skin tissue, even when achieved by contacting the skin surface with a plate or other object cooled to below 0 degrees Celsius, e.g., cooled to about -4 to -10 degrees Celsius, may not always lead to local tissue freezing. Such exemplary cooling procedures can instead lead to locally supercooled tissue that does not freeze. Providing the dimples 210 on a cold contact surface 140, as described in the various exemplary embodiments herein, can increase the likelihood of initiating some local freezing for a particular contact surface temperature and contact time. It can also be desirable in certain instances to be able to confirm the presence of local freezing of skin tissue.

Accordingly, in further exemplary embodiments of the present disclosure, an arrangement can be provided to detect local freezing of skin tissue proximal to the contact surface 140. Such freezing detection can be based on, e.g., a change in optical, thermal and/or mechanical properties detected in the cooled skin, or other indicators and techniques.

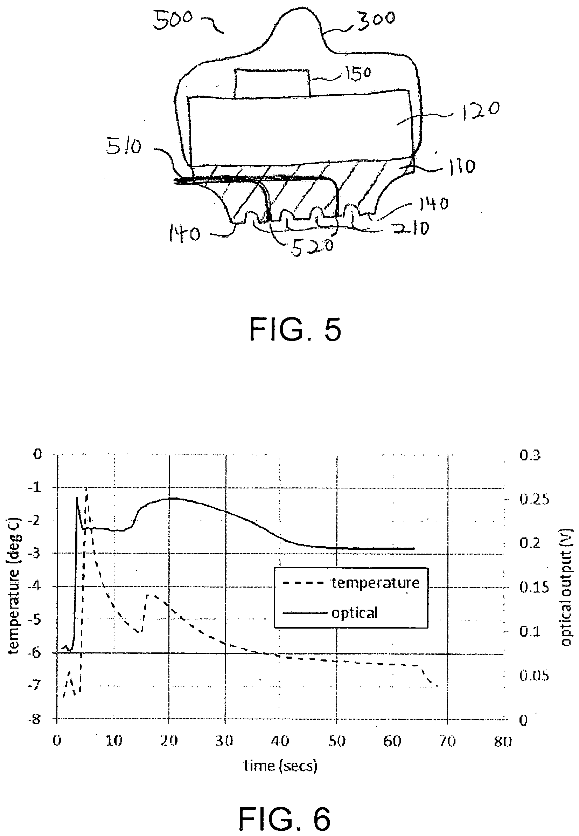

An exemplary apparatus 500 that can facilitate detection of tissue freezing during the cooling procedure according to an exemplary embodiment of the present disclosure is illustrated in FIG. 5. The exemplary apparatus 500 can include a cooling arrangement 120 and an optional cooling plate 110 provided on a lower surface of a cooling arrangement 120, and a handle 130, e.g., similar to the apparatus 100 shown in FIG. 1. The cooling arrangement 120 can include any cooling device or arrangement (or combination thereof) described in the various exemplary embodiments herein. For example, the cooling arrangement 120 can include a Peltier device or the like. One or more optical conduits 510 can be provided with the apparatus 500, where distal ends 520 of the optical conduits 510 can be located proximal to the lower contact surface 140 of the cooling plate 110 (or, optionally, a bottom surface of the cooling arrangement 120 if a separate cooling plate 110 is not provided). The optical conduit 510 can include, e.g., an optical fiber, a waveguide, or the like.

In certain exemplary embodiments of the present disclosure, the distal end 520 of one or more of the optical conduits 510 can be provided proximal to the lower contact surface 140 of the cooling plate 110, e.g., by locating the distal portion of an optical conduit 510 within a small hole drilled through a portion of the bottom plate 110. Other exemplary configurations of the optical conduits 510 can also be provided in further exemplary embodiments, where the distal ends 520 of the optical conduits 510 can be provided proximal to the skin surface and/or in optical communication with the skin surface (e.g., such that there is an unimpeded optical path between the distal end 520 of the optical conduit 510 and the skin surface) when the contact surface 140 of the apparatus 500 is placed against the skin.

To detect freezing of the skin tissue during cooling procedures as described herein, the proximal end of at least one optical conduit 510 can be provided in communication with a source of light or other optical energy (not shown). LEDs or other light sources that emit red light having a wavelength between about 600 nm and about 800 nm can be used as the light source(s). Other wavelengths of light can also be used according to further exemplary embodiments of the present disclosure. For example, light sources that emit light in the near-IR range (e.g., light having a wavelength between about 800 nm and about 2000 nm) can also be used. Such near-IR light may be relatively insensitive to variations in melanin levels of the skin region being treated, and thereby can be used to detect freezing in a variety of skin types.

During the cooling procedure, light can be emitted from the distal end 520 of the at least one optical conduit 510 onto a region of the skin surface beneath the apparatus 500. Light that is reflected and/or scattered by the skin can enter the distal end 520 of at least one optical conduit 510 and directed through the optical conduit 510 to an optical detector (not shown), e.g., a conventional light meter, charge-coupled device (CCD), optical transistor or the like, which can be provided at a proximal end of the optical conduit 510. In further exemplary embodiments of the present disclosure, cladding can be removed from other portions of the optical conduit 510 instead of or in addition to at the distal end thereof, to facilitate detection of light directed toward a circumferential portion of the optical conduit 510, thereby facilitating a detection of light using other orientations of the optical conduit 510 relative to the skin.

A variation in the intensity or other characteristic of the optical light can indicate the occurrence of local tissue freezing. For example, in certain exemplary embodiments of the present disclosure, the optical conduit 510 used to detect scattered and/or reflected light can be the same or similar as the optical conduit 510 used to direct light onto the skin surface, or positioned very close to the optical conduit 510 used to direct light, e.g., within about 1-2 mm. If tissue freezing occurs, a local reflectance of impinging light can increase the amount of light received by the detecting optical conduit 510. Such exemplary increase in an optical signal can be used to confirm freezing of skin tissue when the apparatus 500 is placed on the skin for a particular duration. In further exemplary embodiments of the present disclosure, the optical conduit 510 can be provided with one or more polarizing elements to reduce or suppress specular reflections from the fiber end and tissue surface, which can provide a more sensitive detection of local tissue freezing.

In further exemplary embodiments of the present disclosure, the distal end(s) 520 of the optical conduit(s) 510 used to provide and/or detect light can be spaced further apart, e.g., at a separation distance greater than about 3-4 mm. If tissue freezing occurs, the reflectance of the tissue can increase and more light directed at the skin can be reflected back from the surface region, whereas less light will be scattered laterally through the tissue. Accordingly, a decrease in the detected light signal from such a more distant detecting optical conduit 510 can also indicate a local freezing of the skin tissue. In still further exemplary embodiments of the present disclosure, the distal end 520 of one or more optical conduits 510 can be provided at an inner surface of a dimple 210, instead of or in addition to being provided at a region of the contact surface 140 between the dimples 210 as shown in FIG. 5.

Different exemplary configurations of optical conduits 510 can be provided according to further exemplary embodiments of the present disclosure to detect tissue freezing and/or to obtain more detail about the tissue freezing as described herein. For example, each of a plurality of the optical conduits 510 can be configured to both direct light onto the skin surface beneath the apparatus and detect light scattered or reflected by the skin tissue. A plurality of such optical conduits 510 (e.g., three or more) can be used to provide information on the depth of freezing of the tissue. Alternatively or in addition, a plurality of spaced-apart optical conduits 510 can be configured to direct light onto the skin and/or to detect light as described herein. The optical detector can be configured and/or calibrated to detect a threshold change in the optical signal level that indicates freezing proximal to the distal ends of one or more of the optical conduits 510. In certain exemplary embodiments of the present disclosure, an indicator can be provided, e.g., an LED or light bulb, a sound generator, a digital display, or the like, to confirm the occurrence of tissue freezing while the apparatus 500 is held or otherwise maintained in contact with the skin surface.

In further exemplary embodiments of the present disclosure, the temperature sensors 410, e.g., such as those illustrated in FIG. 4A, can be configured to detect the occurrence of the local tissue freezing. For example, in a typical cooling procedure, the temperature detected by a temperature sensor 410 can correspond to the temperature of the cooling plate 110 that it is in contact with. When the exemplary apparatus 100, 500 is placed on the skin surface, the detected temperature will initially rise as the contact surface 140 of the cooling plate 110 is warmed slightly by the skin. As cooling of the skin by the plate 110 proceeds, the measured temperature can then be decreased. The rate and extent of such decrease can depend on several factors, e.g., the initial temperature, material, and geometry of the cooling plate 110 (including that of the contact surface 140), the efficiency of the cooling arrangement 120 used to cool the plate 110, etc.

Freezing of water (such as that which may be present in skin tissue) is an exothermic process that produces or releases a certain amount of latent heat during the phase transformation from liquid to solid. When tissue freezing occurs proximal to the bottom surface 140 of the cooling plate 110, a slight temporary increase in local temperature may be detected that arises from the release of this latent heat during the freezing phase transformation. The detected temperature may then continue to decrease as further cooling of the (partially-)frozen tissue proceeds. Accordingly, a "bump" detected in the temporal cooling curve by a temperature sensor 410 can also indicate the occurrence of local tissue freezing.

An exemplary study was performed to illustrate the use of optical sensors to detect the onset of freezing in tissue in accordance with embodiments of the present invention. A 20 mm x 20 mm flat aluminum contact plate was cooled to a temperature of -7.5 degrees Celsius. Two 1-mm optical fibers were inserted into holes drilled in the plate, with one fiber configured to illuminate the cooled tissue and the second fiber configured to detect light from the tissue as described herein. The cooled plate was placed in contact with a shaved skin surface on the flank region of a female Sinclair pig for 60 seconds. A thermocouple was used to monitor the temperature at a contact point of the cooling plate and the skin surface. The detected light signal was also monitored and recorded during this cooling procedure.

An exemplary set of data for this cooling procedure is shown in FIG. 6. The measured temperature (indicated by a dashed line in FIG. 6) was initially observed to rise rapidly (at about 5-6 seconds) when the initially cooled plate was brought into contact with the warmer skin surface. The temperature then dropped as the plate cooled the adjacent skin by thermal conduction. This cooling occurred between about 6 and 15 seconds in the data graph shown in FIG. 6. The optical output (measured in volts using an optical transistor, and indicated by a solid line in the graph of FIG. 6) remained fairly constant during this cooling process. At about 15 seconds, a small rise in temperature was detected, indicating the onset of local tissue freezing and release of a latent heat of freezing of the skin. This onset of tissue freezing was accompanied by an increase in the detected optical signal. The optical signal remained elevated as the frozen skin continued to cool. After the cooling plate was removed, it was confirmed that a portion of the surface region of the skin was frozen. This study demonstrates the use of optical sensors as described herein to detect the onset of tissue freezing based on a change in detected reflectance level when a skin surface is contacted by a cold object, and the use of temperature sensors to detect the onset of tissue freezing by the presence of a small plateau or transient rise in local temperature associated with a release of latent heat upon freezing.

In still further exemplary embodiments of the present disclosure, sensors can be provided to facilitate detection of tissue freezing based on measurement of electrical or mechanical impedance. For example, electrical impedance has been shown to change with the onset of freezing in a number of materials including water and tissue. See, e.g., A.A. Gage, Cryobiology 16, pp. 56-62 (1979), B. Rubinsky, Ann. Rev. Biomed. Eng. 02, pp. 157-87 (2000), and T.H. Yu et al., Intl. J. Thermophysics, 24(2) (March 2003). In one exemplary embodiment of the present disclosure, the electrical impedance can be measured between two or more locations along the bottom surface of the cooling plate 110 that is in contact with the skin surface. Alternatively, separate surface electrodes can be provided proximal to the cooled region and used to measure local electrical impedance of the skin. Such sensors for detecting electrical impedance of the skin can be used with any of the embodiments described herein. Any of these types of sensors (thermal, optical, mechanical, force, etc.) configured to detect tissue freezing can be used, either alone or in any combination, with any of the various embodiments of the invention described herein.

In yet further exemplary embodiments of the present disclosure, an indicator can be provided in communication with any of the sensing arrangements (e.g., temperature sensor 410 or optical guide 510) and apparatuses described herein that can be used to detect tissue freezing. Such an indicator can include, for example, an indicator light, a buzzer or other sound generator, a display panel, or the like. The indicator can be configured to provide or otherwise transmit a first signal to a user to indicate when tissue freezing has been detected. Such a signal can optionally be used to determine the start of the desired treatment time. In certain exemplary embodiments of the present disclosure, the sensors can provide signals to the control arrangement 150 to affect operation and operating parameters of the cooling arrangement 120. For example, the control arrangement 150 can transmit a signal or provide a mechanical configuration to turn the cooling arrangement 120 on or off, vary the rate of supplied cooling, etc. based on such signals.

A timing arrangement can also be provided to indicate the time elapsed since the onset of local tissue freezing has been detected. Optionally, the timing arrangement can be configured to provide or otherwise transmit a second signal when a predetermined or preprogrammed time interval has passed since the onset of local freezing. This second signal can be used to indicate when the local cooling treatment has ended, and prompt a user to separate the apparatus used from contacting the skin surface. In further exemplary embodiments of the present disclosure, the timing arrangement may only provide the second signal indicating when the apparatus should be removed from the skin, and not provide the first signal indicating the onset of local freezing.

In still further exemplary embodiments of the present disclosure, the contact element 110 can be formed from two or more materials that can have different thermal effusivities. Such exemplary composite structures can be used to provide particular temperature profiles within the contact element 110, improve temperature controllability of the contact element surface, modify the thermal absorption properties over the surface of the contact element 110, etc. Such two or more materials can be provided as discrete layers or regions of single materials, as a continuous compositional variation, or as a combination of both discrete and continuous combinations of two or more materials.

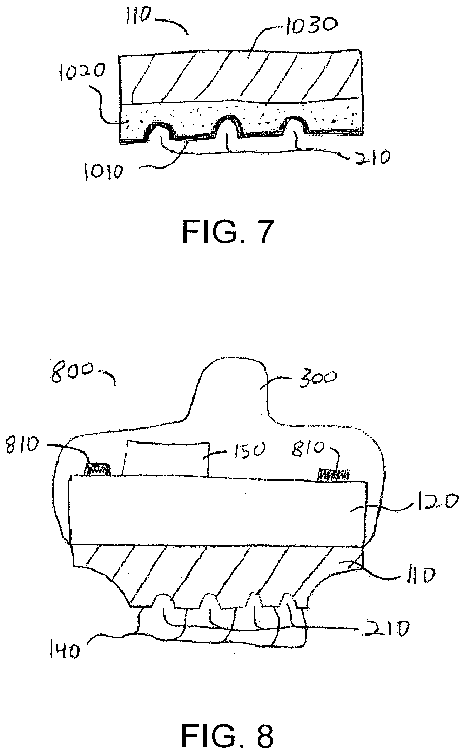

In certain exemplary embodiments of the present disclosure, a material 710 having a thermal effusivity different than that of the upper portion 720 of the contact element 110 can be optionally provided on at least a portion of the distal surface 140 of the contact element 110, optionally in a form of a layer or coating, as shown in the exemplary cross-sectional configuration of FIG. 7. For example, diamond and diamond-like carbon have very high thermal effusivities, and a layer of one of these materials on the lower portion of the contact element 110 may improve thermal transfer between the contact element 110 and the skin being treated. Alternatively, in further exemplary embodiments of the present disclosure, the upper portion 720 of the contact element 110 can have a greater thermal effusivity than the material 710 provided on the distal or lower surface thereof. Such exemplary configuration can further increase the rate of heat extraction of skin tissue 300 protruding into the dimples 210 (as shown in FIG. 3A) relative to the heat extraction rate from skin tissue 300 adjacent to the contact surface 140 between the dimples 210.

In still further exemplary embodiments of the present disclosure, patterns or regions of different materials having different thermal effusivities may be provided within the contact element 110 and/or on the contact surface 140 thereof. Such exemplary variations of the thermal effusivity can be provided, for example, to affect a pattern of heat extraction (e.g. cooling) by the exemplary apparatus 100, 500, to affect or improve temperature control of such exemplary apparatus 100, 500, etc.

In further exemplary embodiments of the present disclosure, an apparatus 800 for affecting tissue pigmentation can be provided that includes one or more pressure sensors 810, as shown in the exemplary cross-sectional configuration of FIG. 8. The exemplary apparatus 800 can also include a handle 300, a cooling arrangement 120, and an optional cooling plate 110 provided on a lower surface of a cooling arrangement 120, e.g., similar to the apparatus 100, 500 shown in FIGS. 1 and 5, respectively, and any variations thereof described herein. The pressure sensor(s) 810 can be used to detect, e.g., a contact pressure between the contact surface 140 of the apparatus 800 and the skin or tissue surface during operation of the apparatus 800. Such pressure detection can be useful, e.g., to ensure a sufficient or appropriate pressure is applied to facilitate good thermal contact between the contact element 110 and the tissue being treated, to indicate whether there is sufficient pressure to promote protrusion of the pliable skin tissue 300 at least partially into the dimples 210 as shown in FIG. 3A, etc. A contact pressure of a few PSI or more (e.g., greater than the systolic pressure in a blood vessel, for example about 17.2 kPa (2.5 PSI) or more) can also produce some local blanching or restriction of blood flow near the tissue surface. Local blanching can reduce the heat transfer to the local tissue by flowing blood, and thereby improve the cooling or heat extraction by the apparatus 100, 500, 800 near the tissue or skin surface.

The pressure sensor(s) 810 can include any conventional components that can be used to detect pressure such as, e.g., a piezoelectric material, a piezoresistive strain gauge, a capacitive or inductive sensor, etc. One or more pressure sensors 810 can be provided in the apparatus 800. Their number, type and/or locations of the pressure sensors 810 can be selected based on several factors including, e.g., reliability of the detected contact pressure, For example, the pressure sensor 810 can be small, have a low thermal mass and/or have a high thermal conductivity to minimize or avoid any reduction in the heat transfer characteristics of the apparatus 800.

The location of the one or more pressure sensors 810 can be selected to provide an accurate indication of the contact pressure between the contact surface 140 and the skin surface during use of the apparatus 800. For example, one or more pressure sensors 810 can be provided between an upper portion of the cooling arrangement 120 and the handle 300, as shown in FIG. 8, if the cooling arrangement 120 and handle 300 are in good mechanical contact. This exemplary configuration can provide pressure sensing capabilities while avoiding any reduction of the heat flow or transfer between the cooling arrangement 120 and the skin surface. In further exemplary embodiments of the present disclosure, one or more pressure sensors 810 can be provided between the cooling arrangement 120 and the contact element 110, on the contact surface 140 of the contact element 110, within the contact element 110, or any combination of such locations.

A pressure indicator (not shown) can be provided on or near the apparatus 800. Such pressure indicator can include a digital or analog readout of the detected contact pressure, an indicator light that can turn on/off or change color to indicate when the contact pressure is within or outside a particular pressure range or above/below a particular limit, an audible signal, etc. The pressure indicator can be used to provide or otherwise transmit a signal to the operator to ensure the presence of an appropriate contact pressure during use of the apparatus 800. This pressure-sensing feature can be used with any of the exemplary embodiments of the apparatus and method described herein including, e.g., different sizes and/or shapes of the dimples 210 and contact elements 110, different cooling arrangements 120, different sensors to detect tissue freezing and/or temperatures, etc.

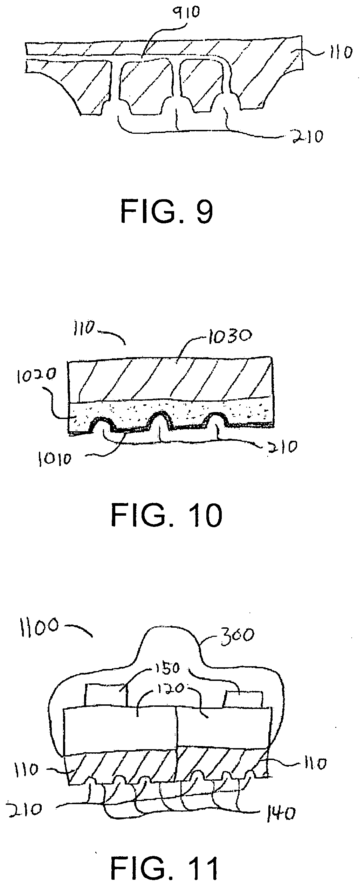

In further exemplary embodiments of the present disclosure, a vacuum arrangement can be used to improve contact between the skin tissue and the contact plate 110, e.g., within the dimples 210. For example, the contact element 110 can include one or more channels 910 therein having a distal end that extends to the lower surface 140 and/or an inner surface of one or more dimples 210, as shown in the exemplary configuration of FIG. 9. A vacuum pump or other conventional source of low pressure (not shown) can be coupled to the proximal end of the channel(s) 910. A valve arrangement (also not shown) can be used to control the presence of a lowered pressure in the channels 910. The channels 910, if present, can be narrow enough to avoid any significant hindrance of heat conduction through the contact element 110. The number, shape, size, and configuration of the channels 910 can be selected based on factors such as the size and shape of the contact element 110, the size, shape and number of dimples 210, etc. The proximal end(s) of the channels 910 can be provided at any location within the apparatus to facilitate connection to a low-pressure source and/or valve arrangement.

Such exemplary vacuum arrangement can facilitate better contact between the contact element 110 and the skin tissue. For example, a low pressure in the channels 910 can result in a lowered pressure in the enclosed volume formed by a dimple 210 and the skin surface, and may facilitate better physical and thermal contact between the interior dimple surface and the skin tissue by drawing the tissue up at least partially into the dimple recess. The exemplary vacuum arrangement can be combined with any of the other various exemplary features and embodiments of the method and apparatus described herein.

In another exemplary embodiment of the present disclosure, the contact surface 140 of the contact element 110 can be flexible or pliable, e.g., to better conform to the local region of skin being treated. For example, an exemplary configuration of a flexible contact element 110 is shown in FIG. 10. The contact element 110 includes a flexible dimpled film 1010, a pliable conductive medium 1020, and an upper thermally conductive layer 1030. In a further exemplary embodiment, the upper thermally conductive layer 1030 may be omitted, such that the conductive medium 1020 is provided in direct contact with the cooling arrangement 120.

The film 1010 can be formed of one or more materials that are flexible or deformable at moderate pressures (e.g., a few PSI or more). Portions of the film 1010 can be sufficiently rigid to maintain a shape of the dimples 210 formed therein. The film 1010 can be formed at least partially of a material having a high thermal effusivity as described herein, e.g., a thermal effusivity that is more than 10 times the thermal effusivity of skin tissue. For example, the film 1010 can be formed at least in part from any one of a variety of metals or metal alloys. Such materials can be sufficiently thin to provide flexibility while also providing structural strength and a high thermal effusivity.

The conductive medium 1020 can include, e.g., a conventional thermally-conductive paste, putty, gel, or the like. In further exemplary embodiments, the conductive medium 1020 can include a two-phase slurry or slush (e.g. a solid/liquid mixture) having a phase change temperature or range that lies within the preferred temperature ranges described herein, e.g., between about -4 and -20 degrees C. Examples of such two-phase mixtures that can be used include, but are not limited to, saline solutions, alcohol/water mixtures, and the like. Such two-phase mixtures can facilitate maintenance of a relatively stable temperature as the apparatus 100, 500, 800 extracts heat from tissue and cools the skin.

The layer or volume of conductive medium 1020 can be relatively thin, as such non-solid materials tend to have a lower thermal diffusivity than certain solid materials such as, e.g., metals or diamond-like carbon. The thickness of the conductive medium 1020 should be large enough to facilitate sufficient deformation of the film 1010 to adapt to the local shape or contour of the skin region being treated. The thickness of such conductive medium 1020 can be selected for a particular apparatus based on, e.g., the desired amount of flexibility or deformation of the film 1010 when the apparatus 100, 500, 800 is in use. Additionally, the contact element 110 having a deformable or flexible contact surface 140 as shown, e.g., in FIG. 10, can be used in conjunction with any of the various embodiments and other features of the exemplary method and apparatus disclosed herein.

In certain exemplary embodiments of the present disclosure, the lower surface of the upper thermally conductive layer 1030 (or the lower surface of the cooling arrangement 120, if the upper conductive layer 1030 is not present) that is in contact with the conductive medium 1020 can be contoured to better conform to the surface contour on which it can be used. For example, the lower surface of the thermally conductive layer 1030 (or the lower surface of the cooling arrangement 120) can be concave or convex, either cylindrically, ellipsoidally, or spherically, or it can have another surface shape or combination of such local shapes, and need not be substantially planar.

In yet another exemplary embodiment of the present disclosure, an apparatus 1100 can be provided that includes, for example, a plurality of cooling arrangements 120 and/or a plurality of contact elements 110 as shown, e.g., in FIG. 11. The exemplary configuration shown in FIG. 11 includes two cooling arrangements 120 and two contact elements 110, with one contact arrangement 110 provided in thermal communication with each cooling arrangement 120. The exemplary apparatus 1100 can also include other combinations of these features such as, e.g., one cooling arrangement 120 provided in thermal communication with two or more contact elements 110, a plurality of cooling arrangements 120 provided in thermal communication with one contact element 110, or combinations of such pairings. For example, a plurality of cooling arrangements 120 can provide better temperature control for one or more contact elements 110 than a single cooling arrangement 120 in certain configurations. Alternatively, a plurality of contact elements 110 can provide contact surfaces 140 that are more adaptive to larger treatment areas of tissue than a single contact element 110 in certain configurations.

Exemplary embodiments of the present disclosure that include a plurality of the cooling arrangements 120 and/or a plurality of the contact elements 110 can be used with any other features or embodiments described herein. For example, one or more of the temperature sensors 410 and/or one or more pressure sensors 810 can be associated with each cooling arrangement 120 and/or with each contact element 110. One or more of the control arrangements 150 (not shown in FIG. 11) can be provided, and certain ones of the temperature sensors 410 and/or pressure sensors 810, if present, can be provided in communication with one or more of such control arrangements 150. Any of the dimple features described herein and illustrated in FIGS. 2B-2K, or any combinations thereof, can be used with any one of the contact elements 110. The design of a particular configuration of the apparatus 1100 that includes more than one of a particular feature can be based on known design principles, e.g., to provide desirable temperature control, facilitate controlled cooling of larger areas of skin tissue, provide variations in local cooling parameters over the entire contact surface 140 of the apparatus 1100, etc. One or more of such contact elements 110 can include a deformable contact film 1010 and a conductive medium 1020 as shown, e.g., in FIG. 10. One or more of the contact elements 110 can also include a plurality of materials having different thermal diffusivities as shown, e.g., in FIG. 7 and described herein.

Multiple cryotherapy treatments in accordance with any of the exemplary embodiments described herein can be performed on a particular area of the skin to produce further lightening of the skin. Such exemplary multiple treatments can be performed at longer intervals, e.g., days or weeks, to allow hypopigmentation effects from a particular treatment to become visually evident before subsequent treatments are performed. Such exemplary multiple treatments can be used to gradually lighten the skin appearance in the treated area.

In further exemplary embodiments of the present disclosure, a method can be provided for gradually and controllably lightening the appearance of skin tissue by generating hypopigmentation using cryogenic techniques. For example, a region of the epidermal skin tissue can be cooled or frozen by contacting it with a contact surface 140 that can include a plurality of dimples 210 as also described herein, e.g., to induce the hypopigmentation using the exemplary temperatures and times described herein. Portions of the upper dermal layer beneath the frozen epidermal regions can also be frozen or cooled during such exemplary procedures. These exemplary time and temperature exposures can induce a hypopigmentation response in the cooled or frozen skin tissue. Accordingly, the exemplary embodiments of the cryogenic hypopigmentation method(s) and/or procedures according to the present disclosure described herein can provide a gradual lightening of the area of skin being treated.

In addition, the exemplary procedures described herein can be repeated on a particular area of skin to further lighten it. Preferably, the interval between successive procedures can be long enough to allow the hypopigmentation effects to be visibly evident and to better control the overall degree of lightening obtained. Multiple exemplary procedures can also be repeated at shorter intervals if desired, for example, to provide a greater overall degree of hypopigmentation in a first area of skin as compared to a second area, using a particular exemplary apparatus to provide contact cooling or freezing at one or more particular temperatures for one or more particular treatment times.

Various combinations of the cooling arrangements, temperature and/or freezing detectors, and other apparatus features described herein can also be used in further exemplary embodiments of the present disclosure, even where certain exemplary combinations may not be explicitly illustrated or described herein in single embodiments. The advantages or characteristics of each of the features can be combined where possible to provide even more advantageous exemplary embodiments.

### EXAMPLE

An exemplary apparatus in accordance with exemplary embodiments of the present disclosure, similar to the apparatus 100 shown in FIG. 1, was used to test the efficacy of the controlled cooling procedure described herein in affecting skin pigmentation. The apparatus includes a thermoelectric chiller having a width of the square cold surface of about 40 mm, and a contact element 110 formed of aluminum that is about 20 mm deep and tapers to a circular contact surface 140 that has a diameter of about 10 mm. The contact surface 140 includes 18 divots 210, where each divot 210 is round in shape with a diameter of 1 mm and a depth of 1 mm. Accordingly, the fractional of the contact surface area covered by divots 210 is about 0.18 (18%). A thermistor was provided in a small groove near an edge of the contact surface 140, such that a side of the thermistor was substantially flush with the contact surface 140. A conventional control arrangement was used for the chiller to maintain the temperature measured by the thermistor at a preset value. A circulating coolant was used to cool the hot side of the chiller. For comparison purposes, a second contact element having similar dimensions, with a slightly convex contact surface and no dimples, was also used to assess the effects of dimples on pigmentation effects for various combinations of contact temperatures and times.

The exemplary apparatus was used to controllably cool marked locations on the flanks of a Sinclair pig for various combinations of contact surface temperatures and contact times. The porcine model is a well-recognized model for biomedical research with physiological and anatomical similarities to the human in many areas. For example, it has been generally recognized that the histological and biochemical properties of pig skin are similar to those of human skin. Like humans, swine are relatively hairless with fixed skin tightly attached to subcutaneous tissue, and pig skin exhibits further similarities to human skin, such as the thickness of the stratum corneum and the follicular structure.

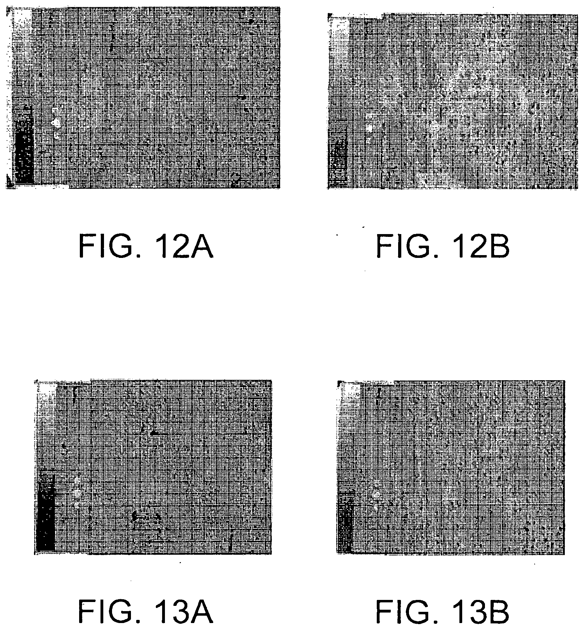

FIG. 12A shows the hypopigmentation that was observed by contacting a particular location of the swine skin in the center of this figure with the dimpled contact surface provided at a temperature of -9 degrees C for a contact duration of 15 seconds, photographed 4 weeks after treatment. FIG. 12B shows the same circular region of hypopigmentation of FIG. 12A that was observed 9.5 weeks after treatment. The lightening of the treated area can clearly be seen in these images.

The central portion of FIG. 13A is a location of the swine skin that was treated by contacting it with the smooth (dimple-less) contact surface, also provided at a temperature of -9 degrees C for a contact duration of 15 seconds, observed 4 weeks after treatment. FIG. 13B shows the same cooled circular region of skin of FIG. 13A 9.5 weeks after treatment. There is little or no discernible lightening of the treated area in these images.

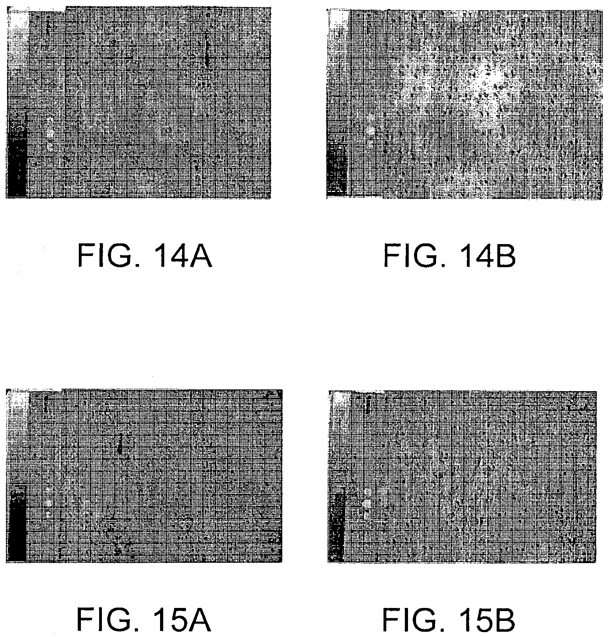

FIGS. 14A and 14B show images of a skin region of the pig (obtained at 4 and 9.5 weeks after treatment, respectively) that was controllably cooled using the dimpled contact surface, with parameters being the same as those described above for FIGS. 12A and 12B, except that the contact duration was 30 seconds. Similarly, FIGS. 15A and 15B show images of a skin region of the pig (obtained at 4 and 9.5 weeks after treatment, respectively) that was controllably cooled using the smooth contact surface, with other parameters being the same as those described above for FIGS. 14A and 14B (e.g., contact duration of 30 seconds at a contact surface temperature of -9 degrees C). Again, little or no hypopigmentation of the treated area can be seen in the treated central portion of these images.

Freezing and subsequent hypopigmentation of skin tissue was observed over a range of time/temperature conditions, including those described for FIGS. 12A-B and 15A-B, when using a dimpled contact surface. In contrast, results were not as consistent when using a smooth contact surface under similar conditions. For example, freezing and subsequent hypopigmentation of skin tissue was not observed when cooling the skin to -9 degrees C for 15 seconds with a smooth contact surface, and only some of the cooled regions of skin exhibited freezing and hypopigmentation when cooled by the smooth contact surface to -9 degrees C for 30 seconds.

In general, it was observed that a dimpled surface can consistently generate some skin freezing and subsequent hypopigmentation in the swine skin under various time/temperature conditions as described herein including, e.g., at -7 degrees C for 15 seconds and -5 degrees for 60 seconds. In contrast, the production of hypopigmentation in skin was not as consistent when using a smooth contact surface under similar time/temperature conditions, and did not produce any hypopigmentation under certain combinations of shorter contact times and slightly warmer temperatures (e.g., -9 degrees C for 15 seconds).

Accordingly, dimpled surfaces can reliably and repeatably initiate freezing and generate hypopigmentation effects in skin tissue for combinations of contact times and temperatures described herein, whereas cooling the skin surface under similar conditions with a smooth cold surface can produce less reliable results. Further, the use of a dimpled contact surface can facilitate production of hypopigmentation at slightly warmer temperatures and/or shorter contact times than can be achieved with a smooth contact surface.

The foregoing merely illustrates the principles of the exemplary embodiments of the present disclosure. Other variations to the disclosed exemplary embodiments can be understood and effected by those skilled in the art in practising the claimed disclosure from a study of the drawings, the disclosure, and the appended claims. A single processor or other unit may fulfil the functions of several items or steps recited in the claims. The mere fact that certain measures are recited in mutually different dependent claims does not indicate that a combination of these measures cannot be used advantageously. Any reference signs in the claims should not be construed as limiting the scope of the claims. Various modifications and alterations to the described exemplary embodiments will be apparent to those skilled in the art in view of the teachings herein. It will thus be appreciated that those skilled in the art will be able to devise numerous techniques which, although not explicitly described herein, embody the principles of the present disclosure and are thus within the scope of the present disclosure.

## Claims

1. An apparatus (100, 500, 800, 1100) for cooling a skin tissue, comprising:
a contact arrangement (110) comprising a distal surface (140) configured to make a continuous contact with a surface of the skin tissue; and
a cooling arrangement (120),
wherein the cooling arrangement is configured to cool the distal surface of the contact arrangement to a temperature of -4 degrees Celsius or colder,
wherein the distal surface of the contact arrangement comprises a plurality of dimples (210) provided therein, the plurality of dimples (210) forming indentations within the distal surface (140), each of the inner surfaces of the indentations being shaped to produce a full contact of the indentations with a corresponding portion of the surface of the skin tissue when the distal surface (140) is placed against the surface of the skin tissue, and
wherein at least one of the plurality of dimples (210) is (i) separated from another one of the plurality of dimples (210) by a particular distance, and (ii) configured to locally deform the skin tissue to provide an increased local surface area of the skin tissue that is in contact with inner surfaces of the plurality of dimples (210).

2. The apparatus of claim 1, wherein a width of each of the indentations is between 0.3 mm and 2 mm, and a depth of each of the indentations is between 0.3 mm and 2 mm.

3. The apparatus of any one of claims 1-2, wherein an area fraction of the indentations on the distal surface of the contact arrangement is between 0.05 and 0.5.

4. The apparatus of any one of claims 1-3, further comprising at least one pressure sensor (810) configured to detect a contact pressure between the distal surface of the contact arrangement and the surface of the skin tissue.

5. The apparatus of any one of claims 1-4, wherein the distal surface of the contact arrangement is flexible.

6. The apparatus of any of claims 1-5, wherein the contact arrangement comprises a proximal end that is configured to contact the cooling arrangement, and wherein the contact arrangement tapers down from the proximal end to the distal surface such that the distal surface of the contact arrangement is smaller in area than the proximal end.

7. The apparatus of any one of claims 1-5, whereineach of the indentations has (i) rounded or beveled edges, and (ii) a hemispherical shape, a cylindrical shape or an ellipsoidal shape.

8. The apparatus of any of claims 1-7, wherein the contact arrangement is configured to couple with the surface of the skin tissue using a pliable, thermally conductive medium, the pliable conductive medium comprising a liquid.

9. The apparatus of any of claims 1-8, wherein the contact arrangement is formed from two or more materials that have different thermal effusivities, wherein an upper portion of the contact arrangement comprises a first material having a first thermal effusivity, and the distal surface of the contact arrangement comprises a pattern of a second material having a second thermal effusivity that is less than the first thermal effusivity.

10. A system for cooling the skin tissue, the system comprising the apparatus of any one of claims 1-9, wherein the contact arrangement is detachable from the cooling arrangement, and the system further comprising a plurality of contact arrangements having different sizes, shapes, and/or surface features.

11. A cosmetic method for lightning an overall complexion or color of a region of a skin, the cosmetic method comprising:
contacting at least one portion of a surface of the region of the skin with a distal surface (140) of a non-penetrating cold object (100, 500, 800, 1100) to make a continuous contact with the surface of the region of the skin, and
cooling the surface of the region of the skin to produce the lightning of the overall complexion or color of the region of the skin,
wherein the distal surface of the cold object comprises a plurality of dimples (210) provided therein, the plurality of dimples (210) forming indentations within the distal surface,
wherein the distal surface of the cold object is provided at a temperature that is between -4 and -20 degrees Celsius,
wherein the distal surface of the cold object is contacted with the skin tissue for less than two minutes, and
wherein the contacting of the surface of the region of the skin with the distal surface of the cold object locally deforms the region of the skin to provide an increased local surface area of the skin tissue that is in contact with inner surfaces of the indentations and produces a full contact of each of the inner surfaces of the indentations with a corresponding portion of the surface of the region of the skin.

12. The method of claim 11, wherein a width of each of the indentations is between 0.3 mm and 2 mm, and a depth of each of the indentations is between 0.3 mm and 2 mm.

13. The method of any one of claims 11 or 12, wherein an area fraction of the indentations on the distal surface of the cold object is between 0.05 and 0.5.

14. The method of any one of claims 11-13, further comprising maintaining a contact pressure between the cold object and the surface of the region of the skin above a predetermined value.

15. The method of any of claims 11-14, wherein the cold object is coupled with the surface of the region of the skin through a pliable, thermally conductive medium, the pliable conducive medium comprising a liquid.

## Patentansprüche

1. Vorrichtung (100, 500, 800, 1100) zum Kühlen eines Hautgewebes, aufweisend:
eine Kontaktanordnung (110), die eine distale Oberfläche (140) aufweist, die so konfiguriert ist, dass sie einen durchgehenden Kontakt mit einer Oberfläche des Hautgewebes herstellt; und
eine Kühlanordnung (120),
wobei die Kühlanordnung so konfiguriert ist, dass sie die distale Oberfläche der Kontaktanordnung auf eine Temperatur von -4 °C oder kälter kühlt,
wobei die distale Oberfläche der Kontaktanordnung in sich mehrere Vertiefungen (210) aufweist, wobei die mehreren Vertiefungen (210) Eindrückungen in der distalen Oberfläche (140) bilden, wobei jede der inneren Oberflächen der Eindrückungen so geformt ist, dass ein vollständiger Kontakt der Eindrückungen mit einem entsprechenden Abschnitt der Oberfläche des Hautgewebes erzeugt wird, wenn die distale Oberfläche (140) gegen die Oberfläche des Hautgewebes angeordnet wird, und
wobei zumindest eine der mehreren Vertiefungen (210) (i) von einer anderen der mehreren Vertiefungen (210) um eine bestimmte Entfernung getrennt ist, und (ii) so konfiguriert ist, dass lokal das Hautgewebe verformt wird, um eine vergrößerte Fläche der Oberfläche des Hautgewebes bereitzustellen, die in Kontakt mit den inneren Oberflächen der mehreren Vertiefungen (210) steht.

2. Vorrichtung nach Anspruch 1, wobei eine Breite jeder der Eindrückungen zwischen 0,3 mm und 2 mm liegt, und eine Tiefe der Eindrückungen zwischen 0,3 mm und 2 mm liegt.

3. Vorrichtung nach einem der Ansprüche 1 bis 2, wobei ein Flächenbruchteil der Eindrückungen auf der distalen Oberfläche der Kontaktanordnung zwischen 0,05 und 0,5 liegt.

4. Vorrichtung nach einem der Ansprüche 1 bis 3, welche weiterhin zumindest einen Drucksensor (810) aufweist, der so konfiguriert ist, dass er einen Kontaktdruck zwischen der distalen Oberfläche der Kontaktanordnung und der Oberfläche des Hautgewebes detektiert.

5. Vorrichtung nach einem der Ansprüche 1 bis 4, wobei die distale Oberfläche der Kontaktanordnung flexibel ist.

6. Vorrichtung nach einem der Ansprüche 1 bis 5, wobei die Kontaktanordnung ein proximales Ende aufweist, das so konfiguriert ist, dass es in Kontakt mit der Kühlanordnung gelangt, und wobei die Kontaktanordnung sich vom proximalen Ende zur distalen Oberfläche verjüngt, so dass die distale Oberfläche der Kontaktanordnung eine kleinere Fläche aufweist als das proximale Ende.

7. Vorrichtung nach einem der Ansprüche 1 bis 5, wobei jede der Eindrückungen (i) abgerundete oder abgeschrägte Ränder aufweist, und (ii) eine halbkugelige Form, eine zylindrische Form oder eine ellipsenartige Form aufweist.

8. Vorrichtung nach einem der Ansprüche 1 bis 7, wobei die Kontaktanordnung so konfiguriert ist, dass sie mit der Oberfläche des Hautgewebes unter Verwendung eines nachgiebigen, wärmeleitfähigen Mediums koppelt, wobei das nachgiebige, leitende Medium eine Flüssigkeit aufweist.

9. Vorrichtung nach einem der Ansprüche 1 bis 8, wobei die Kontaktanordnung aus zwei oder mehr Materialien besteht, die unterschiedliche Wärmeeindringkoeffizienten aufweisen, wobei ein oberer Abschnitt der Kontaktanordnung ein erstes Material mit einem ersten Wärmeeindringkoeffizienten aufweist, und die distale Oberfläche der Kontaktanordnung ein Muster aus einem zweiten Material aufweist, das einen zweiten Wärmeeindringkoeffizienten hat, der niedriger ist als der erste Wärmeeindringkoeffizient.

10. Ein System zum Kühlen des Hautgewebes, wobei das System die Vorrichtung nach einem der Ansprüche 1 bis 9 aufweist, wobei die Kontaktanordnung von der Kühlanordnung abnehmbar ist, und das System weiterhin mehrere Kontaktanordnungen aufweist, die unterschiedliche Abmessungen, Formen, und/oder Oberflächenmerkmale aufweisen.

11. Kosmetisches Verfahren zum Aufhellen einer Gesamt-Hautfarbe oder Farbe eines Bereichs einer Haut, wobei das kosmetische Verfahren aufweist:
Kontaktieren zumindest eines Abschnitts einer Oberfläche des Bereichs der Haut mit einer distalen Oberfläche (140) eines nicht-eindringenden kalten Objektes (100, 500, 800, 1100), um einen durchgehenden Kontakt mit der Oberfläche des Bereichs der Haut herzustellen, und Kühlen der Oberfläche des Bereichs der Haut, um die Aufhellung der Gesamt-Hautfarbe oder Farbe des Bereichs der Haut zu erzeugen,
wobei die distale Oberfläche des kalten Objekts mehrere in ihr vorgesehene Vertiefungen (210) aufweist, wobei die mehreren Vertiefungen (210) Eindrückungen innerhalb der distalen Oberfläche bilden,
wobei die distale Oberfläche des kalten Objekts auf einer Temperatur, die zwischen -4 und - 20 °C liegt, bereitgestellt wird,
wobei die distale Oberfläche des kalten Objekts mit dem Hautgewebe über weniger als zwei Minuten kontaktiert wird, und
wobei das Kontaktieren der Oberfläche des Bereichs der Haut mit der distalen Oberfläche des kalten Objekts den Bereich der Haut lokal verformt, um eine vergrößerte lokale Flächenbereich des Hautgewebes bereitzustellen, die in Kontakt mit inneren Oberflächen der Eindrückungen steht, und einen vollständigen Kontakt jeder der inneren Oberflächen der Eindrückungen mit einem entsprechenden Abschnitt der Oberfläche des Bereichs der Haut zu erzeugen.

12. Verfahren nach Anspruch 11, wobei eine Breite jeder der Eindrückungen zwischen 0,3 mm und 2 mm liegt, und eine Tiefe jeder der Eindrückungen zwischen 0,3 mm und 2 mm liegt.

13. Verfahren nach einem der Ansprüche 11 oder 12, wobei ein Flächenbruchteil der Eindrückungen auf der distalen Oberfläche zwischen 0,05 und 0,5 liegt.

14. Verfahren nach einem der Ansprüche 11 bis 13, weiterhin aufweisend ein Aufrechterhalten eines Kontaktdrucks zwischen dem kalten Objekt und der Oberfläche des Bereichs der Haut oberhalb eines vorbestimmten Wertes gehalten wird.

15. Verfahren nach einem der Ansprüche 11 bis 14, wobei das kalte Objekt mit der Oberfläche des Bereichs der Haut über ein nachgiebiges, wärmeleitfähiges Medium gekoppelt wird, wobei das nachgiebige leitfähige Medium eine Flüssigkeit aufweist.

## Revendications

1. Appareil (100, 500, 800, 1100) pour le refroidissement d'un tissu cutané, comprenant :
un système de contact (110) comprenant une surface distale (140) conçue pour établir un contact continu avec une surface du tissu cutané ; et
un système de refroidissement (120),
le système de refroidissement étant conçu pour refroidir la surface distale du système de contact à une température de - 4 degrés Celsius ou plus froide,
la surface distale du système de contact comprenant une pluralité de fossettes (210) pratiquées dedans, la pluralité de fossettes (210) formant des indentations dans la surface distale (140), chacune des surfaces intérieures des indentations étant conformée pour établir un contact complet des indentations avec une section correspondante de la surface du tissu cutané lorsque la surface distale (140) est placée contre la surface du tissu cutané, et
dans lequel au moins une de la pluralité de fossettes (210) est (i) séparée d'une autre de la pluralité de fossettes (210) par une distance particulière, et (ii) conçue pour déformer localement le tissu cutané pour créer une zone de surface locale accrue du tissu cutané qui est en contact avec des surfaces intérieures de la pluralité de fossettes (210).

2. Appareil selon la revendication 1, dans lequel une largeur de chacune des indentations est comprise entre 0,3 mm et 2 mm, et une profondeur de chacune des indentations est comprise entre 0,3 mm et 2 mm.

3. Appareil selon l'une quelconque des revendications 1 à 2, dans lequel une fraction de surface des indentations sur la surface distale du système de contact est comprise entre 0,05 et 0,5.

4. Appareil selon l'une quelconque des revendications 1 à 3, comprenant en outre au moins un capteur de pression (810) conçu pour détecter une pression de contact entre la surface distale du système de contact et la surface du tissu cutané.

5. Appareil selon l'une quelconque des revendications 1 à 4, dans lequel la surface distale du système de contact est souple.

6. Appareil selon l'une quelconque des revendications 1 à 5, dans lequel le système de contact comprend une extrémité proximale qui est conçue pour venir en contact avec le système de refroidissement, et le système de contact se rétrécit depuis l'extrémité proximale jusqu'à la surface distale, de sorte que la surface distale du système de contact est de surface plus réduite que l'extrémité proximale.

7. Appareil selon l'une quelconque des revendications 1 à 5, dans lequel chacune des indentations a (i) des bords arrondis ou en biseau, et (ii) une forme hémisphérique, une forme cylindrique ou une forme ellipsoïdale.

8. Appareil selon l'une quelconque des revendications 1 à 7, dans lequel le système de contact est conçu pour se coupler à la surface du tissu cutané en utilisant un fluide thermoconducteur pliable, le fluide thermoconducteur pliable contenant un liquide.

9. Appareil selon l'une quelconque des revendications 1 à 8, dans lequel le système de contact est formé de deux matériaux ou plus qui ont des effusivités thermiques différentes, une partie supérieure du système de contact comprenant un premier matériau ayant une première effusivité thermique, et la surface distale du système de contact comprenant un modèle d'un second matériau ayant une seconde effusivité thermique qui est inférieure à la première effusivité thermique.

10. Système de refroidissement du tissu cutané, ce système comprenant l'appareil selon l'une quelconque des revendications 1 à 9, le système de contact étant détachable du système de refroidissement, et le système comprenant en outre une pluralité de systèmes de contact ayant des tailles, des formes et/ou des caractéristiques de surface différentes.

11. Procédé cosmétique éclaircissement d'un teint ou une couleur générale d'une zone d'une peau, ce procédé cosmétique comprenant :
la mise en contact d'au moins une section d'une surface de la zone de la peau avec une surface distale (140) d'un objet froid non pénétrant (100, 500, 800, 1100) pour établir un contact continu avec la surface de la zone de la peau, et
le refroidissement de la surface de la zone de la peau pour produire l'éclaircissement du teint ou de la couleur générale de la zone de la peau,
la surface distale de l'objet froid comprenant une pluralité de fossettes (210) pratiquées dedans, la pluralité de fossettes (210) formant des indentations dans la surface distale,
la surface distale de l'objet froid étant prévue à une température qui est comprise entre -4 et -20 degrés Celsius,
la surface distale de l'objet froid étant en contact avec le tissu cutané pendant moins de deux minutes, et
le contact de la surface de la zone de la peau avec la surface distale de l'objet froid déformant localement la zone de la peau pour créer une zone de surface locale accrue du tissu cutané qui est en contact avec des surfaces intérieures des indentations est établit un contact total de chacune des surfaces intérieures des indentations avec une partie correspondante de la surface de la zone de la peau.

12. Procédé selon la revendication 11, dans lequel une largeur de chacune des indentations est comprise entre 0,3 mm et 2 mm et une profondeur de chacune des indentations est comprise entre 0,3 mm et 2 mm.

13. Procédé selon l'une quelconque des revendications 11 ou 12, dans lequel une fraction de zone des indentations sur la surface distale de l'objet froid est comprise entre 0,05 et 0,5.

14. Procédé selon l'une quelconque des revendications 11 à 13, comprenant en outre le maintien d'une pression de contact entre l'objet froid et la surface de la zone de la peau au-dessus d'une valeur prédéterminée.

15. Procédé selon l'une quelconque des revendications 11 à 14, dans lequel l'objet froid est couplé à la surface de la zone de la peau par un fluide pliable thermoconducteur, le fluide conducteur pliable contenant un liquide.
